# EUROPÄISCHE PATENTANMELDUNG

(11) **EP 2 550 959 A1**
(43) Veröffentlichungstag der Anmeldung: **30.01.2013**
(21) Anmeldenummer: 11075179.9
(22) Anmeldetag: 27.07.2011
(51) Int. Cl.: A61K 9/19, A61K 31/48, A61K 9/00, A61K 9/70, A61P 9/12

(54) **Lisurid, Tergurid und Derivate davon zur Verwendung in der Prophylaxe und/oder Therapie fibrotischer Veränderungen**

(71) Anmelder: Sinoxa Pharma, 14195 Berlin (DE)
(72) Erfinder: Die Erfindernennung liegt noch nicht vor

(57) **Zusammenfassung**

Die vorliegende Erfindung beschreibt die Verwendung von 5-HT2- Rezeptor- Antagonisten und besonders von 8-α-Ergolinen wie Lisurid, Tergurid und deren Derivaten als 5-HT_{2B}- und 5-HT_{2A}-Rezeptor-Antagonisten und Antioxidantien in bevorzugt höher dosierter und bevorzugt kontinuierlicher Anwendung zur Therapie, Progressionsprophylaxe und generellen Prophylaxe von Organfibrosen und anderen, durch mesenchymale Proliferation verursachten pathologischem Organumbau.

## Beschreibung

Gegenstand der vorliegenden Erfindung ist Lisurid, Tergurid und Derivate der allgemeinen Formel (I) 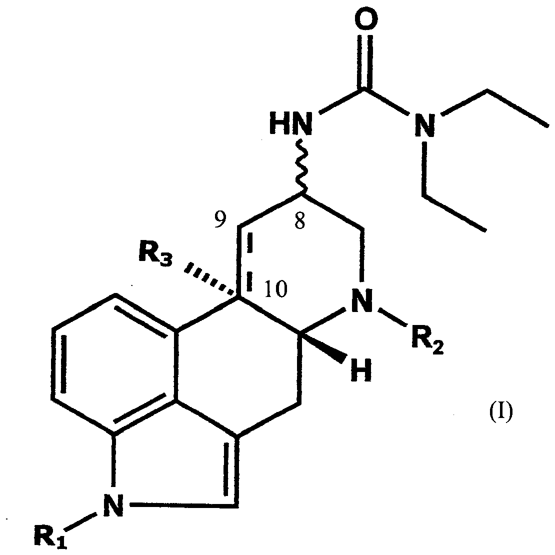
- R1:: allyl, alkinyl
- R2:: ethyl, n-propyl, i-propyl, allyl
- R3:: hydrogen, methyl, ethyl, n-propyl, i-propyl,-CH2OH
wobei die Bindung zwischen C9/C10 entweder eine Einfach- oder eine Doppelbindung ist, zur Verwendung in der Prophylaxe und/oder Therapie fibrotischer Veränderungen von Organen und deren Gefäßstruktur in einem menschlichen oder tierischen Lebewesen zum Aufhalten und / oder zur Rückbildung dieser fibrotischen Veränderungen von Organen und deren Gefäßstruktur. Desweiteren sind pharmazeutische Formulierungen sowie besondere Applikationen dieser pharmazeutischen Formulierungen Gegenstand der vorliegenden Erfindung.

### Hintergrund der Erfindung

Es gibt eine Reihe von Erkrankungen, bei welchen es durch eine Proliferation von Bindegewebszellen und anderen Zellen mesenchymaler Herkunft zu pathologischen fibrotischen und sklerosierenden Veränderungen von Organen und Organsystemen mit oder ohne Kollagenablagerungen und pathologischen Änderungen von Organstruktur und - funktion kommt. Hierzu gehören vor allem fibrotische Organveränderungen mit oder ohne vermehrter Kollagenbildung und -ablagerung, wie sie bei Systemerkrankungen oder Infektionen (z.B. durch HIV, Aspergillus, Mykobakterien, Parasiten) gefunden werden. Primär fibrosierende pathologische Veränderungen finden sich in vielen Organen (z.B. Leberfibrose, Glomerulosklerose, Sklerodermie, Lungenfibrose vielfältiger Genese, andere restriktive Lungenerkrankungen, retroperitoneale und pleurale Fibrosen), zum Teil auch mit vermehrter Kollagenproduktion und -ablagerung als Folge von 5-HT-(=Serotonin)-induzierten trophischen Effekten. Ebenso gehören dazu alle Formen von erhöhtem pulmonalem Gefäßdruck, z.B. als Folge restriktiver oder obstruktiver Lungenerkrankungen (z.B. chronisch-obstruktive Lungenerkrankung (COPD)) sowie die Rechtsherzhypertrophie als Folge von erhöhtem pulmonalem Druck. 5-HT kann diese Veränderungen direkt (z.B. freigesetzt aus Carcinoid-Tumoren) oder sekundär auslösen (z. B. als Folge von Thrombocytenaggregation anderer Ursache im betroffenen Gewebe mit lokaler 5-HT-Anreicherung und -Freisetzung)

Die genannten Erkrankungen sprechen als Folge des fibrosierenden Prozesses auch nicht oder nur sehr begrenzt auf regional und/oder systemisch vasodilatierende Medikamente an, wie die bekannten pulmonalen Vasodilatoren (Prostacycline, Endothelin-Antagonisten, Phosphodiesterase 5-Hemmer) und systemischen Vasodilatoren. Vor allem die oben genannten pulmonalen Vasodilatoren verschlechtern sogar nicht selten das Krankheitsbild [Ulrich-Somaini S, 2009]. Alle diese bisher für die symptomatische Therapie verwendeten Produkte haben auch z.T. ganz erhebliche Nebenwirkungen.

Im Rahmen der vorliegenden Erfindung wurde nun überraschenderweise gefunden, dass die Kombination von hoher 5-HT2B- und 5-HT2A-Rezeptor-Affinität mit starker antioxidativer Wirkung die größten therapeutischen Effekte aufweist. Die größte Wirksamkeit entfalten die erfindungsgemäßen Verbindungen in einer kontinuierlichen Applikation und daraus resultierendem möglichst konstantem Medikamentenspiegel. Lisurid und Tergurid sowie deren Derivate sind bekannt für die Anwendung in der Therapie des pulmonalen arteriellen Hochdrucks (PAH), der Glomerulosklerose und des sekundären Raynaud-Syndroms. Nicht bekannt jedoch waren die Effekte, die auftreten durch die hier vorgestellte Kombination von hoher 5-HT2B- und 5-HT2A-Rezeptor-Affinität im Zusammenhang mit der antioxidativen Wirkung in denselben Molekülen. Dadurch ergeben sich ganz neue Therapiefelder und Applikationen.

Gegenstand der vorliegenden Erfindung sind somit Lisurid, Tergurid und Derivate von Lisurid und Tergurid mit der allgemeinen Formel (I) 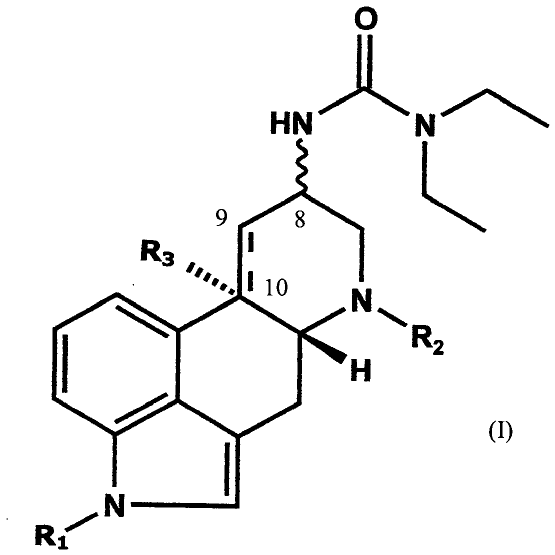
- R1:: allyl, alkinyl
- R2:: ethyl, n-propyl, i-propyl, allyl
- R3:: hydrogen, methyl, ethyl, n-propyl, i-propyl,-CH2OH
wobei die Bindung zwischen C9/C10 entweder eine Einfach- oder eine Doppelbindung ist, zur Verwendung in der Prophylaxe und/oder Therapie fibrotischer Veränderungen von Organen und deren Gefäßstruktur in einem menschlichen oder tierischen Lebewesen zum Aufhalten und / oder zur Rückbildung dieser fibrotischen Veränderungen von Organen und deren Gefäßstruktur.

Eingeschlossen als Gegenstand der vorliegenden Erfindung sind insbesondere sowohl 8α-Enantiomere als auch 8β-Enantiomere der erfindungsgemäßen Verbindungen gemäß der allgemeinen Formel I. Insbesondere sind somit 8α-Lisurid und 8α-Tersurid sowie 8β -Lisurid und 8β -Tergurid Gegenstand der vorliegenden Erfindung. Dies ist überraschend, da für die bekannte dopaminerge Wirkung der Substanzen die alpha-Konfiguration entscheidend ist. Eine Veränderung der Konfiguration in der 8-Position zur beta-Konfiguration bedeutet einen Verlust der dopaminergen Wirkung. Im Hinblick auf die erfindungsgemäße Wirkung am 5-HT2B-und 5-HT2A-Rezeptor sind jedoch beide Konfigurationen wirksam, siehe Tabelle 3, Beispiel 11. Dies kann die Verträglichkeit dieser Therapien bei fibrosierenden Erkrankungen deutlich verbessern.

Die Begriffe "Fibrotische Veränderungen von Organen und deren Gefäßstruktur" umfassen fibrotische Veränderungen von Organen und /oder Organsystemen sowie pathologische Strukturveränderungen von Organen und /oder Organsystemen durch mesenchymale Proliferation, gebräuchlich ist auch der Begriff Organfibrose.

Überraschenderweise führt die erfindungsgemäße Verwendung von Lisurid, Tergurid und Derivaten der allgemeinen Formel (I) in der Prophylaxe und/oder Therapie zur Lebensverlängerung des Lebewesens. Bisherige Therapien waren bisher lediglich geeignet, die Symptome von fibrotischen Erkrankungen zu lindern. Die Therapie mit den erfindungsgemäßen Verbindungen führt jedoch zu einer echten Besserung durch das Aufhalten beziehungsweise die Rückbildung der fibrotischen Veränderungen und damit zu einer Verlängerung der Lebenserwartung.

Lebewesen im Sinne der Erfindung sind Säugetiere, insbesondere menschliche Lebewesen, also insbesondere Menschen, die z.B. an einer Fibrose erkrankt sind.

Am meisten bevorzugt als therapeutische Substanz und als aktive Substanz in pharmazeutischen Formulierungen ist Lisurid und Derivate von Lisurid der allgemeinen Formel (I), am allermeisten bevorzugt ist Lisurid selbst, sowohl 8α-Lisurid als auch 8β-Lisurid.

Die Verwendung in der Prophylaxe und/oder Therapie der obengenannten Erkrankungen von Lisurid, Tergurid und Derivate der allgemeinen Formel (I) erfolgt bevorzugt in der Weise, dass während der Therapiezeit mindestens 80 % der Zeit, bevorzugt mindestens 100 % der Therapiezeit, die 5-HT_{2B}- und/oder 5-HT_{2A}-Rezeptor-Belegung im Zielorgan am meisten bevorzugt vollständig ist, wenigstens jedoch 90 %, bevorzugt wenigstens 95 %, am meisten bevorzugt 100 % beträgt also vollständig ist. Zielorgan im Sinne der vorliegenden Erfindung ist jedes fibrotische, bindegewebig oder durch sonstiges mesenchymales Wachstum pathologisch verändertes Gewebe im Organismus.

Die Bestimmung der Rezeptordichte kann semiquantitativ oder quantitativ erfolgten und zwar wie folgt:
Lungengewebe wird in einer 4%ige Paraformaldehydlösung fixiert und dann in Paraffin eingebettet. 3micro-m-Schnitte werden für die Immunhistochemie nach Herstellerangaben (Zymed labs./Invitrogen, Carlsbad, Ca., USA) in 6.5 mM Na-citrat (pH 6.0) unter Druck erwärmt und mit Antikörper gegen 5-HT2B-Rezeptor (ab12926 von Abcam, Cambridge, UK ) 1:200 inkubiert und mit dem Vulcan fast red Chromogen-Kit (Zymed) im Schnitt angefärbt und mit Kontrollgewebe verglichen (s.z.B. Dumitrascu et al.,Eur. Resp.J. 37, 1104-1118, 2011).

Quantitatative Reverse-Transcriptase Polymerase Chain Reaction (q RT-PCR) erfolgt mit aus eingefrorenem Lungengewebe isolierter RNA und von dieser induzierter cDNA (Promega, Madison, Wi., USA) im Mx3000P Real-Time PCR System (Stratagene, La Jolla, Ca., USA) und die Rezeptor-RNA wird dann gegen eine Porphobilinogen-Referenz aus demselben Gewebe quantitativ bestimmt (s.z.B. Dumitrascu et al., s.o.).

Mit Therapiezeit ist folgendes gemeint: die Rezeptorblockade muß solange, wie Krankheitssymptome bestehen, über den ganzen Zeitraum, d.h.7 Tage pro Woche und 24 Stunden am Tag, erfolgen. Das heißt, 80 % der Therapiezeit bedeutet z.B. 19,2 Stunden am Tag.

Die außergewöhnlich hohe 5-HT2B- und 5-HT2A-Rezeptoraffinität von Lisurid und seinen Derivaten und deren weitgehend gleichmäßige und rasche Aufnahme ins Gewebe führt zu einer in der Regel vollständigen Rezeptorblockade, wie u.a.Untersuchungen mit radioaktiv markiertem Lisurid zeigen können. Dabei wird das zu untersuchende Gewebe vor bzw. nach einer Lisurid-Behandlung im Versuchsmodell präpariert und homogenisiert, und dann die spezifische Lisurid-Bindung durch eine übliche Messung der Radioaktivität im Szintillationszähler bestimmt. Durch die hohe Rezeptoraffinität kommt es dabei zu einer lokalen Anreicherung dieser Wirkstoffe im pathologisch veränderten Gewebe, zumal dort oft auch die entsprechenden Rezeptoren vermehrt exprimiert sind. Dies mit der dann hiermit verbundenen lokal verstärkten Antioxidationswirkung führt dann auch zu einer sehr spezifischen Wirkung auf die Gewebepathologie. Die Rezeptoren selbst wurden dabei im Gewebe immunhistochemisch visualisiert bzw. mit z.B. RT-PCR auch quantifiziert, wie oben beschrieben.

Hohe 5-HT2B- und 5-HT2A-Rezeptoraffinität im Sinne der vorliegenden Erfindung bedeutet einen pA2 -Wert von 7 (d.h. über dem entsprechenden Wert für den physiologischen Agonisten 5-HT, der am 5-HT2b-Rezeptor bei 6.5 liegt), besser bei 8 und mehr, und bevorzugt bei 9 und noch höher (s. Beispiel 11). Dabei bedeutet der pA2 -Wert den negativ dekadischen Logarithmus der Konzentration eines Antagonisten, die es erforderlich macht, die Agonisten-Konzentration zu verdoppeln, um den Ausgangseffekt des Agonisten ohne Antagonisten wiederherzustellen. Diese Untersuchungen erfolgen entweder an geklonten menschlichen Rezeptoren, die auf CHO-Zellen exprimiert werden (Newman-Tancredi et al., J. Pharmacol. Exper.Ther. 303, 815-822, 2002) oder, wie im Beispiel 11 beschrieben, mit einem funktionellen Rezeptorassay an Pulmonal- bzw. Koronar-Arterien vom Schwein (Görnemann et al., J. Pharmacol. Exp. Ther. 324,1136-1145, 2008).

Die Verwendung in der Prophylaxe und/oder Therapie der obengenannten Erkrankungen von Lisurid, Tergurid und Derivate der allgemeinen Formel (I) erfolgt bevorzugt in der Weise, dass während der gesamten Therapiezeit mehr als 90 % der Zeit, bevorzugt 100 % der Zeit, die 5-HT_{2B}- und/oder 5-HT_{2A}-Rezeptor Belegung im Zielorgan vollständig ist.

Die Verwendung in der Prophylaxe und/oder Therapie der obengenannten Erkrankungen von Lisurid, Tergurid und Derivate der allgemeinen Formel (I) erfolgt des Weiteren bevorzugt in der Weise, dass der Wirkstoffspiegel in der systemischen Zirkulation des Lebewesens während der Therapiezeit mindestens 80 % der Zeit, bevorzugt mindestens wenigstens 90 % am allermeisten bevorzugt 100 % der Zeit (kontinuierlich) wenigstens 5 pg/ml, mehr bevorzugt wenigstens 100 pg/ml, mehr bevorzugt wenigstens 200 pg/ml, am meisten bevorzugt 300 - 500 pg/ml beträgt.

Dies hat eine Ermittlung des Wirkstoffspiegels durch spezifische Bioassays (z.B. LC / MS /MS oder Radioimmunoassay) in pharmakokinetische Untersuchungen an Probanden gezeigt. Daraus ergibt sich, daß eine gute, auf jeden Fall aber für die Therapie ausreichende Korrelation zwischen der infundierten Menge Lisurid und den damit erzeugten Plasmakonzentrationen besteht und damit auch mit der Rezeptorblockade. Auch eine eventuelle Überdosierung ist nicht problematisch, da Lisurid und seine Derivate reine Antagonisten sind ohne jede intrinsische agonistische (d.h. krankheitsbefördernde) Wirkung. Außerdem gibt es ausreichend klinische Ergebnisse, wonach auch höhere Dosierungen, wie sie zur Parkinsontherapie verwendet werden, gut vertragen werden. Diese Überlegungen gelten auch für andere pharmazeutische Formen zur Erreichung ausreichend hoher konstanter Plasma-Spiegel wie etwa transdermale Applikationsformen oder hochdosierte orale Retardformulierungen.

In den genannten pharmakokinetischen Studien an Probanden wurde unverändertes Lisurid im Blutplasma durch ein HPLC-Verfahren mit massenspektrometischer Detektion (LC/MS/MS) selektiv und mit hoher Genauigkeit bestimmt. Die analytischen Bestimmungen wurden mit tert-butyl-methyl-Ether-Extrakten aus Plasmaproben durchgeführt, wobei jeweils 400µl Plasma mit 900µl tbmE, welches den externen Standard Protergurid (2 ng/ml) enthielt, extrahiert wurden.

Der Extrakt wurde nach Evaporation in das organische Laufmittel Acetonitril/Wasser (3β:70)/0,1% Ameisensäure aufgenommen und mit einer Flußrate von 300µl/min an einer C6-Phenyl-Säule mittel Gradientenelution (10mM Ammoniumformiat/0,1% Ameisensäure gegen das genannte organische Laufmittel chromatographiert. Die Detektion erfolgte unter Verwendung eines Massenspektrometers (TSQ) mit Elektrospray (ESI)-Interface. Sensitivität und Selektivität für Lisurid (n=5) wurde mit 9% Std.-Abweichung und einem Signal/Rauschverhältnis von 40 bestimmt bei einer Konzentration von 20 pg/ml. Die Präzision der Methode wurde z.B. bei einer Konzentration von 60 pg/ml mit 3% Std.-Abweichung und die untere quantitative Nachweisgrenze (LLoQ) mit 5 pg/ml bestimmt. Das heißt, dass die oben gemachten Werte und Angaben zum Wirkstoffspiegel in der systemischen Zirkulation des Lebewesens während der Therapiezeit nach einem solchen Verfahren bestimmt werden können.

Die Verabreichung von Lisurid, Tergurid und Derivate der allgemeinen Formel (I) in der Prophylaxe und/oder Therapie der obengenannten Erkrankungen erfolgt bevorzugt in einer Dosis von 0,01 bis 5,0 mg pro Tag, bevorzugt von 0,15 bis 3,0 mg pro Tag am meisten bevorzugt von 0,25 bis 1,0 mg pro Tag erfolgt.

Die Verabreichung von Lisurid, Tergurid und Derivate der allgemeinen Formel (I) in der Prophylaxe und/oder Therapie der obengenannten Erkrankungen erfolgt bevorzugt kontinuierlich, dass heißt, daß der Wirkstoffpegel während der gesamten Therapiezeit möglichst konstant ist beziehungsweise vor allem den oben genannten Wirkstoffpegel während der gesamten Therapiezeit nicht unterschreitet.

Die Verabreichung von Lisurid, Tergurid und Derivate der allgemeinen Formel (I) in der Prophylaxe und/oder Therapie der obengenannten Erkrankungen erfolgt am meisten bevorzugt kontinuierlich in einer Tages-Dosis von 0,01 bis 5,0 mg , bevorzugt von 0,15 bis 3,0 mg am meisten bevorzugt von 0,25 bis 2,0 mg.

Die erfindungsgemäße Verabreichung von Lisurid, Tergurid und Derivate der allgemeinen Formel (I) zur Verwendung in der Prophylaxe und/oder Therapie erfolget in einer der bevorzugten Ausführungsformen an einem Lebewesen, welches an erhöhtem pulmonalen arteriellen Gefäßdruck (PAH) leidet. In einer Ausführungsform der Erfindung ist die der PAH Folge einer Erkrankung ausgewählt aus der Gruppe enthaltend COPD, Infektionen, Rechtsherzhypertrophie, -insuffizienz als Folge pulmonaler Hypertension, sowie sonstige fibrotische Veränderungen der Lunge, Leber, Niere, Haut oder anderer Organsysteme.

Gegenstand der vorliegenden Erfindungen sind ebenfalls pharmazeutische Zubereitungen enthaltend Lisurid, Tergurid und Derivate der allgemeinen Formel (I) zur Verwendung in der Prophylaxe und/oder Therapie der oben ausgeführten Erfindungsgegenstände.

Bevorzugt sind pharmazeutische Zubereitungen gemäß der vorliegenden Erfindung, mit einer Einzeldosis von Lisurid oder Tergurid oder Derivaten der allgemeinen Formel (I) im Bereich von 0,01 bis 2.5 mg, und je nach der Schwere der Erkrankung für den Patienten einer Tagesdosis bevorzugt im Bereich von 0,15 bis 3,0 mg, am meisten bevorzugt im Bereich von 0,25 bis 2,0 mg.

Pharmazeutische Zubereitungen können erfindungsgemäß ausgewählt sein aus der Gruppe der Formulierungen enthaltend Tabletten, Schichttabletten, überzogenen Tabletten, Pillen, Weich- oder Hartkapseln, Mikrokapseln, orale Retardarzneiformen, transdermalen Systeme, Suppositorien, micro- und nanokristallinen Formulierungen, liposomalen Formulierungen, Tropfen, Nasentropfen, Sprays, Emulsionen, Dispersionen, Lösungen, sterile Lösungen Lyophilisaten, Pulver und Inhalationssprays.

Die Applikation oder Anwendung der erfindungsgemäßen pharmazeutischen Zubereitung ist bevorzugt ausgewählt aus der Gruppe enthaltend orale, perorale, sublinguale, buccale, subkutane, intravenöse dermale, pulmonale oder nasale Anwendung bzw. Applikation, wobei eine subkutane Anwendung am meisten bevorzugt ist.

Am meisten bevorzugt ist eine sterile Lösung entweder als Lyophilisat zur Zubereitung einer sterilen Lösung vor der Anwendung oder als gebrauchsfertige sterile Lösung mit einer Dosierung von 0,25 bis 1,0 mg für die kontinuierliche vorzugsweise subkutane Infusion mit einer Infusionsrate von 0,05 bis 50 mcg/h vorzugsweise 1 bis 20 mcg/h .

In einer Ausführungsform der Erfindung enthalten pharmazeutische Zubereitungen mindestens eine der beanspruchten Verbindungen insbesondere Lisurid oder Tergurid oder Derivate der allgemeinen Formel (I) in einer Einzeldosis der Wirkstoffe von 0,1 bis 10 mg formuliert mit zumindest einem pharmakologisch kompatibel Hilfsstoff, Lösungsmittel oder Carrier.

Pharmazeutische Zubereitungen werden vorzugsweise ausgeboten als sterile Lösungen bzw. Lyophilisate, parenterale, perorale und orale Retardarzneiformen, transdermale Systeme, mikro- und nano-kristalline Formulierungen, liposomale Formulierungen, Mikrokapseln, Emulsionen, Dispersionen und sind insbesondere geeignet für die subkutane, intravenöse, dermale, transdermale, orale, perorale oder pulmonale Anwendung bzw. Applikation.

Laktose, Stärke, Sorbitol, Mannitol, Sucrose, Ethylalkohol und Wasser können zum Beispiel als pharmakologisch und chemisch kompatibler Carrier, Lösungsmittel, oder Hilfsstoffe verwendet werden.

Weiterhin können Stärke, modifizierte Stärke, Gelatine, natürliche Zucker, natürliche oder synthetische Polymere, wie zum Beispiel Acacia Gummi, Guar, Natriumalginat, Carboxymethylcellulose oder Polyethylenglycol als Bindemittel enthalten sein. Cyclodextrine, modifizierte Cyclodextrine, ebenso Benzoate, Chloride, Acetate, Tartrate können als Stabilisatoren enthalten sein und Stearate, Polyethylenglycol, Aminosäuren wie wie zum Beispiel Leucin können als Hilfsstoffe üblicherweise in Konzentrationen von 0,05 bis 15 % eingesetzt werden.

Flüssige Formulierungen beinhalten Lösungen, Dispersionen und Emulsionen. Flüssige Zubereitungen für die parenterale Anwendung sind steril und enthalten Wasser oder Wasser und Lösungsvermittler, wie zum Beispiel Propylenglycol, Micell- und Mischmizellbildner.

Stärke oder modifizierte Stärke, Alginate, Aluminate, Bentonite oder mikrokristalline Cellulose können als in Konzentrationen üblicherweise zwischen 2 und 30% nach Gewicht eingesetzt werden.

Zucker, Zuckeralkohole, Mais-, Reis-oder Kartoffelstärke Gelatine. Gummi Arabicum, TraganthSugar, Ammoniumcalciumalginate Carboxymethylcellulose, Hydroxypropylmethylcellulose, Polyvinylpyrrolidon auch anorganische Stoffe könne als Hilsstoffe üblicherweise in Konzentrationen zwischen 1 bis 30% nach Gewicht eingesetzt werden.

Pharmazeutische Zubereitungen zur subkutanen, intravenösen und transdermalen Anwendung ebenso wie parenterale und orale Arzneiformen mit modifizierter Freisetzung werden als bevorzugte Formulierungen beansprucht. Solche Formulierungen bestehen in der Regel aus einer Matrix, insbesondere aus einer Matrix mit Polymeren in vielen Fällen bioabbaubare Polymere als formgebenden, konstituierenden Zusatz, in die mindestens eine der beanspruchten Verbindung vorzugsweise Lisuride oder Terguride oder Derivate der Formel (I) eingearbeitet ist.

Die nachstehend aufgeführten Polymere werden als Beispiel für die oben genannten Matrixkonstituierenden Polymere beansprucht: Polyvalerolacton, Polylactide, Polyglycolide, Copolymere der Polylactide und Polyglycolide, Poly-e-Caprolacton, Poly-hydroxybuttersäure Polyhydroxyvalerate, Poly(1,4-dioxan-2,3-dione), Poly(1,3-dioxan-2-one). Polyanhydride wie Polymaleinsäureanhydrid, Polyhydroxymethacrylate, Fibrin, Polycyanoacrylate, Polycaprolactondimethylacrylat, Poly-b-maleinsäure, Polycaprolactonbutyl-acrylat, Multiblock-Polymere wie beispielhaft von Oligocaprolactondiolen und Oligodioxanondiolen, Polyetherester-Multiblock Polymere wie beispielhaft PEG and Poly(butylenterephtalat). Polypivotolactone, Polycaprolacton-Glycolide, Poly(g-Ethylglutamate), Polyorthoester, Polytrimethylcarbonate, Poly-Iminocarbonate, Poly(N-vinyl)-pyrolidone, Polyvinylalcohole, Polyesteramide, glycolierte Polyester, Polyphospho-Ester, Polyphosphazene, Poly[p-Carboxyphenoxy)propan] Polyhydroxypentansäure, Polyanhydride, Polyethylenoxidpropylen oxid, Polyurethane, Polyurethane mit Aminosäure-Reste im Gerüst, Polyetherester wie Polyethylenoxid, Polyalkenoxalate, Polyorthoester und deren Copolymere, Carrageenane, Fibrinogen, Stärke, Protein-basierte Polymere, Polyaminosäuren, synthetische Polyaminosäuren, Zein, modifiziertes Zein, Polyhydroxy-Alkanoate, Pectinsäure, modifiziertes und non-modifiziertes Fibrin und Casein, Carboxymethylsulfat. Albumin, weiterhin Hyaluronsäure, Heparansulphate, Heparin, Chondroitinesulphat, Dextran, Cyclodextrine, Copolymere mit PEG und Polypropylenglycol, Gummi arabicum, Guar, Gelatine, collagen, Collagen-N-Hydroxysuccinimid, Modificationen und Copolymere und/oder Mischungen dieser Substanzen.

Biopolymere sind bevorzugt wie zum Beispiel Stärke and denaturierte Stärke, Cellulose, Glycosaminoglycane and Kollagen wie auch semi-synthetische und synthetische Polymere wie Silicone, Silicon-Elastomere, Polydimethylsiloxan, Polydimethylsiloxan enthaltend Siliciumdioxid, Polydimethylsiloxan enthalten Polyalkylenoxid (Gelest®), Polytetrafluoroethylen (Teflon®), Polylactide, Polyglycolide, Polyethylenglycol. Polylactidpolyglycolid-Co-Polymere, Polyanhydride, Ethylenvinylacetat-Polymere, Poly(methylmethacrylat), Celluloseethylether, Poly(ethyl-acrylat), Poly(trimethylammoniumethyl-methacrylat), Polydimethylsiloxane, Hydroxyethyl-Polymethacrylate, Polyurethane und Polystyren-butadien-Copolymere.

Darüber hinaus können perorale Arzneiformen mit modifizierter Freisetzung wie auch transdermale Systeme Mikrospheren oder Nanopartikel oder Mikrokristalle enthalten oder diese als konstituierenden Bestandteil enthalten und mindestens eine der beanspruchten Verbindung vorzugsweise Tergurid and Lisurid enthalten. Die beanspruchten Partikel oder Kristalle können darüber hinaus in Gele eingebracht werden und in dieser Form appliziert werden, ebenso adhäriert werden an biokompatible Keramikmaterialien wie Hydroapatit.

Überraschenderweise sind gerade bei den beschriebenen fibrotischen Erkrankungen die 8-α-Ergoline Lisurid und Tergurid sowie ihre Derivate wirksam und zwar aufgrund ihres direkten antagonistischen Effekts auf trophische Aktivierung von Fibroblasten, Fibromyoblasten, T-Zellen und anderen Mesenchymzellen, wie sie vor allem durch eine Aktivierung von 5-HT2B-Rezeptoren verursacht werden sowie durch weitere nicht-vaskuläre Mechanismen.

Dabei ist für die gewünschte Hemmung fibrotischen Organumbaus vor allem von Bedeutung, dass bei den erfindungsgemäßen Verbindungen der beschriebene 5-HT2B-antagonistische Effekt überraschend auch kombiniert ist mit starker antioxidativer Wirkung, welche diese Substanzen als hervorragende Radikalfänger auszeichnet. Die Kombination von hoher 5-HT2-Rezeptor-Affinität mit starker antioxidativer Wirkung, wie sie bei Lisurid und seinen Derivaten vorliegt, ist überraschend und insofern von großer Bedeutung, als in kürzlichen Untersuchungen festgestellt wurde, dass z.B. der pathogenetische Prozess der 5-HT2-induzierten Herzhypertrophie unter Generierung von Sauerstoffradikalen abläuft [Bianchi P *et al.,* 2005] und durch Radikalenfänger antagonisiert werden kann [Redout EM *et al.,* 2010]. Serotonin kann aber auch rezeptorunabhängig freie Radikale generieren, wenn es aus Thrombozyten in hoher lokaler Konzentration freigesetzt wird. Insbesondere der neu gefundene kombinierte Effekt, wie oben beschrieben, trägt damit also wesentlich zu einer Hemmung pathologischen Gewebewachstums bei, da er verschiedene pathogenetische Mechanismen simultan inhibiert.

Von Bedeutung ist in diesem Zusammenhang, dass sich diese neuen und überraschenden Wirkungen von Lisurid und seinen Derivaten vor allem durch höher dosierte und möglichst kontinuierliche Wirkstoff-Applikation erreichen lassen. Eine solche Anwendungsweise hat sich bereits als gut verträglich, individuell nach Bedarf adjustierbar und hochwirksam in der kontinuierlichen dopaminergen Stimulation bei fortgeschrittenem M. Parkinson erwiesen [Stocchi F *et al.,* 2002]. Im Fall der oben genannten überraschenden neuen Anwendungsgebiete beruhen die Wirkungen aber nicht auf den bekannten dopaminergen Effekten von Lisurid, sondern auf seiner hohen antagonistischen Wirkung auf 5-HT2B-Rezeptoren [Jaehnichen S *et al.,* 2005] in Kombination mit seinem 5-HT2A-Antagonismus und seiner starken anti-oxidativen Wirkung [Bianchi P *et al.,* 2005].

Diese fibrosierenden und proliferativen pathologischen Organerkrankungen sind dadurch charakterisiert, dass sie primär oder sekundär durch 5-HT (Serotonin) und/oder oxidativen Stress bedingt sind. Sie entstehen vor allem durch die Aktivierung trophischer 5-HT-Rezeptoren (in der Regel Subtypen des 5-HT2-Rezeptors), und oft sind die lokalen 5-HT-Konzentrationen (z.B. aus Thrombozyten) erhöht und/oder die trophischen Rezeptoren vermehrt exprimiert. Wichtig ist dabei auch, dass schon kurze Pulse von erhöhter 5-HT-Ausschüttung (wie z.B. beim Carcinoid Syndrom) und/oder kurze Phasen von oxidativem Stress zu dauerhaftem pathologischem Organumbau mit Schädigung der Organfunktion führen können. Besonders durch die im Fall von Lisurid in anderen Indikationen bereits bewährten Anwendungsformen (z.B. durch sc-Infusionen mit Hilfe tragbarer Minipumpen, durch transdermale therapeutische Systeme, aber auch andere Depotformen) ist es möglich, eine Hemmung der serotonin-induzierten trophischen Aktivierung von Fibroblasten, T-Zellen und anderen mesenchymalen Zellen vollständig und über die gesamte Tages- und Nachtzeit zu gewährleisten und damit ein mögliches "Durchbruch- oder Escape-Phänomen" mit daraus folgender Unwirksamkeit zu verhindern. Ein gleicher Effekt lässt sich auch durch höher dosierte orale Anwendungsformen und Retardformulierungen bei guter Verträglichkeit erreichen, da sich die 5-HT2B- antagonistische Wirksamkeit z.B. von Lisurid schon in deutlich geringerer Konzentration erreichen lässt als die bekannte und zugelassene Anwendung von Lisurid als Dopaminagonist.

Die vorliegende Erfindung beschreibt die Verwendung von 5-HT-2- Rezeptor-Antagonisten und besonders von 8-α-Ergolinen wie Lisurid (CAS-No.:18016-80-3,3-(9,10-didehydro-6-methylergoline-8alpha-yl)-1,1-diethylurea), Tergurid (trans-Dihydrolisurid) und deren Derivaten als 5-HT2B- und 5-HT2A-Rezeptor-Antagonisten und Antioxidantien in höher dosierten und bevorzugt kontinuierlichen Anwendungsformen zur Therapie, Progressionsprophylaxe und generellen Prophylaxe von Organfibrosen und anderem, durch mesenchymale Proliferation verursachtem pathologischem Organumbau. Hierzu gehören vor allem die sekundären Formen von pulmonalem arteriellem Hochdruck, die z.B. nach COPD, Infektionen, Lungenfibrose auftreten können, die Rechtsherzhypertrophie als Folge erhöhten pulmonalen Gefäßdrucks sowie der fibrotische Umbau von Leber, Nieren, Haut oder anderen Organsystemen.

Die Erfindung bezieht sich weiter auf Salze, Enantiomere, Enantiomerengemische, Diastereomere und Diastereomerengemische, Hydrate, Solvate und Racemate der oben aufgeführten Verbindungen für die Herstellung einer pharmazeutischen Zubereitung zur Therapie, Progressionsprophylaxe und generelle Prophylaxe von Organfibrosen und anderen durch Mesenchym-Aktivierung und Kollagenbildung verursachten Organumbau. Hierzu gehören vor allem sekundäre Formen des pulmonalen Hochdrucks, die Rechtsherzhyperthrophie als Folge erhöhten pulmonalen Gefäßdrucks und weitere Organfibrosen, sowie der fibrotische Umbau von Nieren, Leber, Haut oder anderen Organen.

Die beanspruchten Verbindungen Lisurid und Tergurid sind alkalisch und durch Säurezugabe können entsprechende Salze erhalten werden, wobei organische oder anorganische Säuren verwendet werden können. Zu den Säuren, die diese Art Salze der Verbindungen nach der allgemeinen Formel I bilden, gehören Schwefelsäure, Sulfonsäure, Phosphorsäure, Salpetrige Säure, Salpetersäure, Perchlorsäure, Hydrobromsäure, Salzsäure, Ameisensäure, Essigsäure, Propionsäure, Bersteinsäure, Oxalsäure, Glucuronsäure (in links- und rechtsdrehender Form), Milchsäure, Äpfelsäure, Weinsäure, (Hydroxymalonsäure, Hydroxypropandicarbonsäure), Fumarsäure, Zitronensäure, Ascorbinsäure, Maleinsäure, Malonsäure, Hydroxymaleinsäure, Brenztraubensäure, Phenylessigsäure (o-, m-, p-) Toluinsäure, Benzoesäure, p-AminoBenzoesäure, Salicylsäure, p-Amino-Salicylsäure,

Methylsulfonsäure, Ethylsulfonsäure, Hydroxymethylsulfonsäure, Ethylensulfonsäure, p-Toluolsulfonsäure, Naphthylsulfonsäure, Naphthylaminosulfonsäure, Sulfanilsäure, Camphersulfonsäure, Chininsäure, o-Methylmandelsäure, Pikrinsäure, (2,4,6-Trinitrophenol), Adipinsäure, Aminosäuren wie z.B. Methionin, Tryptophane, Arginin, und insbesondere saure Aminosäuren wie Glutamin oder Aspartinsäure.

Wenn Säuresubstituenten in den Verbindungen vorliegen, können auch basische Additionssalze gebildet werden, insbesondere mit Alkalimetallen wie auch mit Aminosäuren. Deshalb können Alkalimetallsalze wie das Natrium-, Kalium-, Lithiumsalz oder das Magnesium-, Calciumsalz, Alkylaminosalze oder Salze mit Aminosäuren gebildet werden z.B mit alkalischen Aminosäuren wie Lysine.

Die beanspruchten Verbindungen Lisurid und Tergurid sind insbesondere geeignet für die Behandlung oder Prophylaxe von pathologischem Organumbau, wie er durch 5-HT und/oder durch lokalen oxidativen Stress verursacht wird.

Die vorliegende Erfindung betrifft die Anwendung des starken und nicht-antagonisierbaren 5-HT2B-Antagonisten Lisurid, seiner Derivate und anderer Moleküle vergleichbarer Wirkung zur Behandlung oder Verhinderung fibrotischer Organveränderungen und zur nachfolgenden Restrukturierung und Normalisierung von betroffenen Organen und Organfunktionen.

Besonders durch die im Fall von Lisurid in anderen Indikationen bereits bewährten Anwendungsformen (z.B. durch sc-Infusionen mit Hilfe tragbarer Minipumpen, durch transdermale therapeutische Systeme, aber auch andere Depotformen) ist es möglich, eine Hemmung der trophischen Aktivierung von Fibroblasten, T-Zellen und anderen mesenchymalen Zellen vollständig und über die gesamte Tages- und Nachtzeit zu gewährleisten und damit eine intermitierende Aktivierung der 5-HT2- Rezeptorsubtypen sowie ein mögliches "Escape-Phänomen" mit daraus folgender Unwirksamkeit der Therapie zu verhindern. Ein gleicher Effekt lässt sich auch durch höher dosierte orale Anwendungsformen und Retardformulierungen bei guter Verträglichkeit erreichen, da sich die 5-HT2B- antagonistische Wirksamkeit z.B. von Lisurid schon in deutlich geringerer Konzentration erreichen lässt als die bekannte und zugelassene Anwendung von Lisurid als Dopaminagonist.

Die oben aufgeführten und beanspruchten Wirkstoffe und Substanzen sind geeignet zur Behandlung, Progressionsprophylaxe und allgemeinen Prophylaxe von Organfibrosen z.B. der Lunge und von anderem durch Mesenchym-Aktivierung und Kollagenbildung verursachtem Organumbau sowie für dessen Normalisierung. Dass die Substanzen der Formel I für diese Anwendung bei Organfibrosen geeignet sind, ist für den Fachmann überraschend. Bisherige Hersteller zugelassener Produkte auf der Basis von Lisurid (z.B. Dopergin®) als Prolaktinsenker und Parkinsonmittel und Tergurid (Teluron® in Japan) warnen sogar vor der Auslösung fibrotischer Organveränderungen wie Herzklappen-, Pleura- oder Pericardfibrose sowie retropleuraler und -peritonealer Fibrose als möglicher unerwünschter Nebenwirkungen dieser Substanzen, wie dies auch in der Tat für Substanzen mit Ergolinstruktur bekannt ist, z.B. für Cabergolin, Pergolid, Ergotamin, Methysergid. Diese Kenntnisse haben den Fachmann bisher von den neu gefundenen Anwendungen der genannten Substanzen abgehalten.

Ein zur Erzielung der gewünschten therapeutischen Wirkung relevanter neuer Befund ist, dass für die pro-fibrotischen Effekte von 5-HT bereits ein sehr kurzes intermittierendes Zeitintervall der Einwirkung ausreicht und dass deshalb eine kontinuierliche Anwendung von 5-HT2B-Antagonisten z.B. mittels tragbarer Minipumpen, transdermale Systeme mit Langzeitfreigabe, Implantate oder orale Retardformen die beschriebenen Organfibrosen am besten verhindern oder in ihrer Progression hemmen können.

Es ist bekannt, dass Lisurid und Tergurid in ihren jetzt zugelassenen Anwendungen häufige und zum Teil auch erhebliche Nebenwirkungen auslösen können. Hierzu gehört z.B. die orthostatische Hypotonie, die zu Kreislaufkollaps und Ohnmacht führen kann und auch mit Aktivitäten des normalen Lebens interferiert. Deshalb wird von den Herstellern auch vor dieser Nebenwirkung gewarnt. Eine solche Nebenwirkung ist beim pulmonalen arteriellen Hochdruck und anderen Organfibrosen äußerst unerwünscht und im Einzelfall auch gefährlich. Auch aus diesem Grund würde ein Fachmann nicht auf den Gedanken kommen, derartige Substanzen in den beschriebenen Indikationen therapeutisch einzusetzen.

Die Auslösung von orthostatischer Hypotonie und Kollaps durch die beschriebenen Substanzen beruht ebenso wie deren bisherige therapeutische Anwendungen auf ihrer bekannten dopaminergen Wirkung. Sie wird ebenso wie andere häufige dopaminerge Nebenwirkungen nicht durch Überschreiten einer kritischen Dosierung oder Plasmakonzentration ausgelöst, sondern wird durch stark schwankende, oszillierende Plasmaspiegel, vor allem bei oraler Einnahme mit rasch erreichten hohen Peaks verursacht. Bei längerer Anwendung kommt es zur Toleranzentwicklung, was die therapeutische Nutzung in den beschriebenen Indikationen erlaubt. Die beschriebenen Nebenwirkungen können aber durch die erfindungsgemäßen kontinuierlichen Formen der Anwendung ohnehin weitestgehend vermieden werden, da es hier nur zu geringen Schwankungen der Plasmaspiegel über die Zeit kommt und auch ein "First-pass-Effekt" in der Leber vermieden wird.

Eine erfindungsgemäße Anwendung von Lisurid, Tergurid und deren Derivaten als 5-HT2B-Antagonisten in den beschriebenen Formen von Organfibrosen und vergleichbaren Erkrankungen des Mesenchyms wird überraschenderweise auch dadurch erleichtert, dass wegen der höheren Affinität der erfindungsgemäßen Verbindungen zum 5-HT-Rezeptor in der Regel niedrigere Dosierungen als für die bekannten Anwendungen als Dopaminagonist therapeutisch wirksam sind. Dies bedeutet, dass die generelle Verträglichkeit dieser Behandlungen noch günstiger wird und sich damit auch deutlich von den bisher verwendeten vasodilatorischen Substanzen unterscheidet. Die Anwendung wird auch durch die in der Regel sehr niedrige Dosierung der beschriebenen 5-HT2B-Antagonisten, deren einfachen Metabolismus und meist auch problemloser individueller Dosierbarkeit sehr erleichtert. Diese Eigenschaften erleichtern auch die oft erforderliche Kombinationstherapie mit anderen Wirkstoffen für dieselbe Indikation oder für Begleitkrankheiten, wobei die sehr einfache Dosisanpassung der Lisuridinfusion ein weiterer Vorteil ist.

Für die beschriebenen Anwendungen zusätzlich günstig sind die weiteren antagonistischen Wirkungen von Lisurid, Tergurid und anderen 5-HT2B-Antagonisten auf andere Subtypen des 5-HT2-Rezeptors und weitere Monoamin-Rezeptoren. So hat z.B. Lisurid als sehr wirksamer peripherer 5-HT2A-Antagonist eine günstige Wirkung auf die durch 5-HT verursachte oder verstärkte Aggregation von Thrombozyten, die indirekt über erhöhte lokale 5-HT-Spiegel gleichfalls 5-HT-Rezeptoren stimulieren oder direkt durch Bildung von freien Radikalen ebenso zu Organfibrosen beitragen können.

Überraschend günstig wirkt sich die sehr hohe Affinität der beschriebenen Substanzen für 5-HT2B Rezeptoren (antagonistische Wirksamkeit bei bis zu 10⁻¹⁰ M Konzentrationen) auch dadurch aus, dass sich so die Wirkstoffe vor allem in den fibrotisierten Organen anreichern können, in denen die 5-HT2-Subrezeptoren oft besonders stark exprimiert sind. Da z.B. ein Molekül Lisurid bis zu 6 freie Sauerstoffradikale aufnehmen kann, wirken die beschriebenen Substanzen so zusätzlich auch über diesen Mechanismus antifibrotisch und anti-entzündlich. Dies erfolgt besonders dort, wo durch verstärkte Rezeptorexpression eine solche antioxidative Wirkung therapeutisch dringend erwünscht ist.

Vorteilhaft ist auch, dass die beschriebenen 5-HT2B-Antagonisten nur den erhöhten arteriellen Blutdruck in der Lunge antagonisieren, nicht aber den systemischen Blutdruck in nennenswertem Umfang beeinflussen. Hoher Blutdruck entsteht entweder durch eine Erkrankung und Verengung der Arterien und der ihnen nachgeschalteten Arteriolen und Kapillaren; dies ist beim arteriosklerotischen systemischen Hochdruck der Fall (wie er mit der üblichen manometrischen Blutdruckmessung bestimmt wird), aber auch beim vaskulär verursachten idiopathischen Hochdruck in der Lunge (hier wird der Gefässdruck über eingeführte Herzkatheter oder indirekt per Echokardiographie bestimmt). Die zweite mögliche Ursache für erhöhten arteriellen Druck liegt in einem erhöhten Widerstand in durchbluteten Organen, wie er z.B. durch Organfibrosen (oder auch im Fall der Niere durch Glomerulosklerose) verursacht wird

Überraschend ist, dass die erfindungsgemässen Substanzen mit 5-HT2B-Antagonismus nicht nur die Fibrose-verursachenden Effekte von 5-HT antagonisieren, sondern auch eine Restrukturierung, d.h. ein erneutes "Re-modeling" pathologischer Organstrukturen bewirken können. Sie fördern damit also den Wiederaufbau normaler Organstruktur und Funktion. Dies betrifft z.B. bei erhöhtem pulmonalem Druck nicht nur die Lungengefäße, sondern auch das rechte Herz. Dabei ist wichtig, dass Patienten mit pulmonaler Hypertension häufig infolge der resultierenden Hypertrophie des rechten Ventrikels und daraus folgender Rechtsherzinsuffizienz früh versterben, sodass der beschriebene therapeutische Effekt der 5-HT2B-Antagonisten lebensverlängernd sein kann. Überraschenderweise ist ein solcher remodeling-Effekt der beschriebenen 5-HT2B-Antagonisten auf das hypertrophierte Herz primär nicht ausschließlich die Folge einer Besserung des überhöhten arteriellen Lungendrucks und der Lungenfibrose und Funktion. Vielmehr handelt es sich dabei auch um einen eigenständigen therapeutischen Effekt der 5-HT2B-Antagonisten auf die durch exzessive 5-HT2B-Stimulierung verursachte Myocardhypertrophie, ein Effekt, der bereits kurze Zeit nach Beginn der Behandlung nachgewiesen werden kann (z.B. mittels Echocardiographie). Es ist möglich, dass dies ontogenetisch eine führende Rolle von 5-HT2B-Rezeptoren beim normalen Aufbau des Herzens in der pränatalen Phase reflektiert.

Neue Untersuchungen von Villeneuve *et al.* [2009] haben am Beispiel der Herzventrikel gezeigt, dass 5-HT, möglicherweise freigesetzt aus Thrombozyten, das pathologische Remodeling des Herzens, wie es dem Herzversagen (und Tod) vorausgeht, in entscheidender Weise, aber auf verschiedenen Wegen auslöst. Dabei unterscheiden Villeneuve et al das Wachstum der Fibroblasten des Herzens, wo über 5-HT2B-Rezeptorenaktivierung hypertrophie-fördernde Zytokine und Interleukine freigesetzt werden, von einer direkten Aktivierung kardialer Myoblasten. Diese Aktivierung durch 5-HT erfolgt über 5-HT2A-Rezeptoren, bei höheren 5-HT-Konzentrationen aber auch durch dessen Aufnahme über einen spezifischen 5-HT-Aufnahme-Mechanismus direkt in diese Zellen, wo 5-HT dann mit Hilfe der Monoaminoxidase A durch die Bildung von freien Radikalen ("reactive oxygen species, ROS") gleichfalls pathologisches Wachstum und Organumbau induziert.

Bei den erfindungsgemässen Substanzen zeigt es sich jetzt überraschend, dass z.B. Lisurid, aber auch Tergurid und deren Derivate neben ihrer starken 5-HT2B-antagonistischen Wirksamkeit in Konzentrationen ähnlicher Grössenordnung auch starke periphere 5-HT2A-Antagonisten sind: damit hemmen sie nicht nur die sekundäre Thrombozytenaggregation [Glusa E *et al.,* 1984] unabhängig von deren Auslösung, sondern auch die direkte Aktivierung und Proliferation der Myoblasten selbst. Hinzukommt, dass diese Substanzen überraschenderweise überaus starke Radikalenfänger sind: so kann ein einzelnes Molekül Lisurid bis zu 6 freie Sauerstoffradikale aufnehmen, Tergurid bis zu 4. Untersuchungen zur 5-HT2-induzierten Herzhypertrophie haben nachgewiesen, dass dieser Prozess unter Generierung von Sauerstoffradikalen abläuft [Bianchi P *et al.,* 2005]. Wenn man weiter berücksichtigt, dass diese Substanzen durch ihre bisher nie erreichte hohe 5-HT2-Rezeptoraffinität bevorzugt an diesen angereichert werden (die wiederum bei Organhypertrophie lokal verstärkt exprimiert sind) so trägt auch diese kombinierte Eigenschaft wesentlich zu einer Hemmung pathologischen Organwachstums bei. Zudem wirken diese Substanzen über alle diese Mechanismen auch entzündungshemmend, sodass sie auch bei entzündlich ausgelöster Organpathologie (z.B. auch bei pulmonalem arteriellem Hochdruck ausgelöst durch COPD oder Infektionen) wirksam sind. Eine derartige Kombination erwünschter Wirkungsmechanismen, wie im Falle der beschriebenen Substanzen, hätte auch ein Fachmann auf diesem Gebiet nicht vorhersehen können. In der Tat haben die erfindungsgemäßen Substanzen die unten aufgezählten Effekte gegen Organfibrosen, Organhypertrophien und pathologischen Organumbau. Hierzu gehören neben den direkten auch indirekte Effekte der 5-HT2B-Rezeptor-Antagonisten mit resultierender anti-fibrotischer und anti-proliferativer Wirkung, wie im Folgenden aufgelistet:

Direkte Effekte der erfindungsgemäßen Substanzen
1. Hemmung von 5-HT2B-Rezeptoraktivierung, wie sie zum Wachstum von Fibroblasten und deren mesenchymalen pathologischen Folgen führen;
2. Hemmung von 5-HT2A-Rezeptoraktivierung, wie sie zu sekundärer Thrombozytenaggregation und daraus resultierender 5-HT-Ausschüttung erfolgt;
3. Hemmung von 5-HT2A-Rezeptoren auf organspezifische Zellen, wie sie z.B. auf Myoblasten vermehrt exprimiert sind und zur pathologischen Organhypertrophie führen;
4. Hemmung der Entstehung und Wirkung freier Sauerstoff-Radikale (ROS), wie sie in einem eigenständigen Mechanismus zur Organhypertrophie führen, als hochwirksame Radikalenfänger.

Indirekte Effekte der erfindungsgemäßen Substanzen
1. Hemmende Effekte auf Re-modeling Prozesse, wie sie aus der Interaktion der 5-HT2B-Rezeptor-Antagonisten mit dem 5-HT-Transporter und hinsichtlich der 5-HT-Clearance in der Lunge entstehen.
2. Hemmung des fibrotischen Organumbaus durch Interaktion der erfindungsgemäßen Substanzen mit pro-fibrotischen Mediatoren wie z.B. PDGF und Zytokinen.

Diese Effekte lassen sich besonders günstig therapeutisch nutzen, indem eine konstante Substanzwirkung (z.B. durch eine kontinuierliche s.c. Infusion) erreicht wird.

### Beispiele

### Beispiel 1

### Pharmakologische Eigenschaften

### 1.A. 5-HT2B Antagonismus von Lisurid und Tergurid

Trophische, über 5-HT2B-signalling mediierte Wirkungen von Serotonin sind in einer Reihe von Zelltypen nachgewiesen worden, hauptsächlich in Fibroblasten. Sie sind verantwortlich für exzessive vaskuläre Re-modeling-Prozesse und Organumbau. Für verschiedene mit der Auslösung von pathologischen Herzklappenveränderungen und pulmonaler Hypertension assoziierte Verbindungen ist belegt, dass der Organumbau als Folge der Aktivierung des 5-HT2B-Rezeptors zustande kommt, entweder direkt oder über aktive Metaboliten. Zu diesen Verbindungen gehören Pergolid, Cabergolin, Fenfluramine (über den aktiven Metaboliten Methyl-Ergonovin), MDA und MDMA (ecstasy), Bromocriptin, Methysergide (über den aktiven Metaboliten methyl-ergonovine) und Ergotamin. Innerhalb der Klasse der Ergoline scheint die entscheidende Determinante für den Agonismus am 5-HT2B-Rezeptor in der β-Orientierung des 8-Substituenten zu liegen.

### 1. B. 5-HT2A Antagonismus von Lisurid und Tergurid

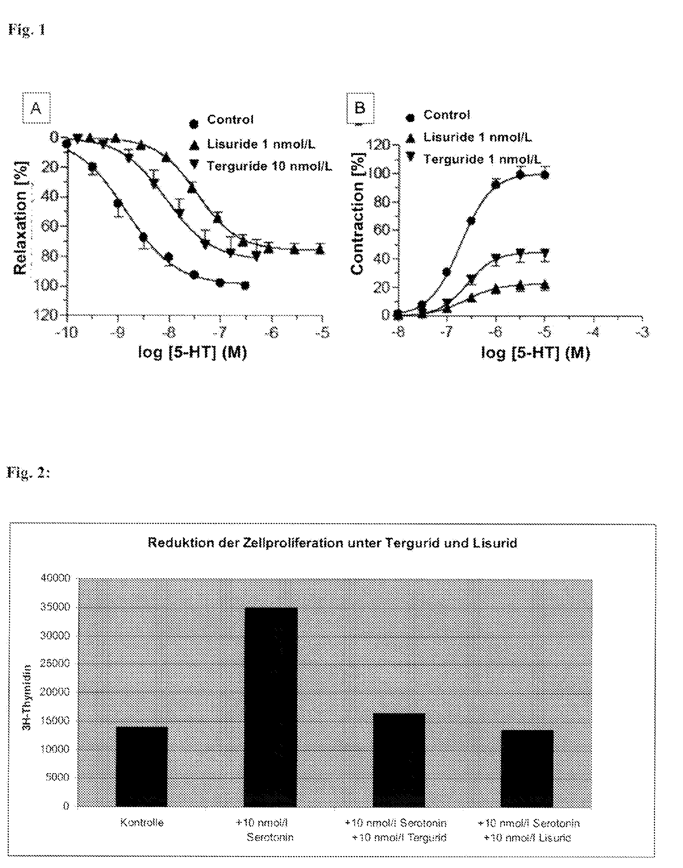

Aktivierung von HT2A-Rezeptoren führt zu thrombozyten-aggregierenden und vasokonstriktiven Effekten und wird mit pro-thrombotischen und hypo-fibrinolytischen Prozessen in Verbindung gebracht. Die Hemmung der 5-HT2A-mediierten Kontraktion der Koronararterien des Schweins wurde benutzt, um die Interaktion von Lisurid mit 5-HT2A-Rezeptoren zu charakterisieren. In diesem Modell hat Lisurid auch in hohen Konzentrationen keine intrinsische agonistische Aktivität. Dagegen hemmt Lisurid in Gegenwart von 5-HT die Vasokonstriktion mit einer IC50 von 1 nmol/L (siehe Fig. 1B).

Bewertung des Experimentes: Lisurid und sein Derivat Tergurid weisen ein sehr ähnliches pharmakologisches Profil auf mit identischer, wenn auch schwächerer Aktivität von Tergurid an den entscheidenden 5-HT2- Rezeptorsubtypen.

### 1.C. Anti-serotoninerge Eigenschaften von Lisurid und Tergurid

Eine besonders hohe anti-serotoninerge Potenz wurde für Lisurid in vitro am isolierten Magen der Ratte nachgewiesen, wo 5-HT die 5-HT2B-Rezeptoren aktiviert [Villalon *et al.* 2003] und ebenso in Tiermodellen für Hyperserotoninämie und 5-HT-induzierte Effekte [Podvalova I *et al.,* 1972]. Ebenso unterdrückte Tergurid Verhaltensabnormalitäten und fibrotische Hautveränderungen an den Injektionsorten von 5-HT bei der Ratte. Im gleichen Experiment führte die tägliche Gabe von 5-HT über 4 Monate bei einer Reihe von Ratten zur Entwicklung einer pulmonalen Klappeninsuffizienz, während dies bei den mit Tergurid behandelten Tieren nicht nachweisbar war. Analog verhinderte Tergurid den 5-HT-induzierten Gewichtsanstieg von Herz und Leber [Hauso O *et al.,* 2007].

Da Lisurid und sein Derivat Tergurid ein sehr ähnliches pharmakologisches Profil aufweisen mit identischer, wenn auch schwächerer Aktivität von Tergurid an den entscheidenden 5-HT2- Rezeptorsubtypen, können diesbezügliche, bei Tergurid gefundene Effekte auch auf Lisurid extrapoliert werden. Bewertung der Experimente: Unter physiologischen Bedingungen ist Lisurid ein nicht-kompetitiver Antagonist des 5-HT2A-Rezeptors und ein irreversibler Antagonist des 5-HT2B-Rezeptors, das heißt, er kann selbst durch höchste 5-HT-Konzentrationen nicht antagonisiert werden. Die vaskuläre Reaktion in Präparationen der Pulmonalarterien des Schweins, die mittels PGF2α vorkontrahiert waren, wurden als Assay für die spezifische Interaktion mit 5-HT2B-Rezeptoren verwendet (siehe Fig. 1A). Lisuride hemmt 5-HT-Effekte in pico- und nanomolaren Konzentrationen. Das steht in guter Relation mit der EC50 von 5-HT für eine Gefäßrelaxierung über Aktivierung endothelialer 5-HT2B-Rezeptoren.

### Beispiel 2

### Antiproliferative Wirkung von Lisurid und Tergurid

Menschliche glatte Muskelzellen mesenterialen Ursprungs (Promo Cell) wurden entsprechend den Empfehlungen des Herstellers bis zur Bildung geschlossener Monolayers in 6 Platten mit PromoCell-Kulturmedium vermehrt und dann im gleichen Medium auf 24-Vertiefungsträgern für eine Zellzahl von 5x104 Zellen/Ansatz ausgesät. Das Zellwachstum wurde dann mittels Zugabe von 10-8 mol/l 5-HT-stimuliert. Zur Bestimmung des Zellwachstums wurde dann 3H-Thymidin (Amersham) zu den Zellkulturen hinzugegeben und diese für 24 Stunden inkubiert. Nach der Adhäsion der Zellen wurde das Kulturmedium durch ein Standardmedium mit 0.2% fetalem Kalbsserum zum Wachstumsstopp ersetzt und erneut 48 Stunden lang inkubiert.

Um antiproliferative Wirkungen der beschriebenen Substanzen zu testen, wurden die Zellkulturen dann zunächst mit einer Konzentration von 10 µmol/l der Prüfsubstanzen präinkubiert. Das Zellwachstum wurde dann durch Zugabe von 5-HT bis zu einer Endkonzentration von 10-8 mol/l stimuliert. Zur Messung des Wachstums der Zellen wurde dann 3H-Thymidin (Amersham) den Kulturen zugefügt und diese für 24 Stunden inkubiert. Danach erfolgten 2 Inkubationen in eisgekühlter Kochsalzlösung mit einem Phosphat-Puffer und danach 30 min Inkubation in eisgekühlter 10%iger Trichlor-Essigsäure bei 4°C. Die Zellen wurden dann in 0.1 molarer NaOH-Lösung inkubiert (0.5 ml/Inkubationsgefäss). Nach Neutralisierung mit Essigsäure wurde die 3H -Thymidin-Aufnahme mit Flüssig-Szintillation gemessen (als Dreifach-Bestimmung). Die gefundenen Mittelwerte finden sich in der Fig.2.

Bewertung des Experiments:
Die Ergebnisse zeigen, dass das durch 5-HT über trophische 5-HT2B-Rezeptoren induzierte Wachstum mesenchymaler Zellen der menschlichen Lunge (glatte Muskelzellen aus Lungengefässen und Bronchien bzw. Alveolen, aber wohl auch Bindegewebs-Fibroblasten) durch die beschriebenen 5-HT2B-Antagonisten rasch und effektiv gehemmt wird. Es ist hervorzuheben, dass in diesem Modell mit menschlichen Zellen keine vasodilatorischen oder Endotheleffekte eine Rolle spielen können, Mechanismen, wie sie zur Zeit im Falle von Tergurid bei idiopathischem pulmonalem Hochdruck klinisch geprüft werden; vielmehr zeigt sich hier überraschend eine primäre Wirkung der beschriebenen Substanzen auf die Proliferation mesenchymaler Zellen.

Zusammengefasst beschreibt dieses Beispiel, dass sich 5-HT2B-Antagonisten zur Behandlung von Zuständen krankhaft gesteigerter Zellproliferation und zur gewerblichen und funktionalen Restrukturierung von Organen bei nicht-idiopathischem pulmonalem Hochdruck und anderen Organfibrosen eignen.

### Beispiel 3

### Pulmonaler Hochdruck

Es wurden die Wirkungen von Lisurid und Tergurid beim monocrotalin-induzierten Lungenhochdruck der Rate untersucht [Reiter R et al, 2007]. Monocrotalin (MCT) ist ein Toxin aus Pflanzen der Spezies Crotalaria, welches nach einer einzigen Injektion in Ratten die Endothelzellen der pulmonalen Arterien schädigt mit folgender Hypertrophie der glatten Gefässmuskulatur und persistierendem schwerem pulmonalem Hochdruck. Die MCTinduzierte pulmonale Hypertension der Ratte ist ein gut etabliertes und validiertes Modell der humanen pulmonalen Hypertension und alle heute zugelassenen Therapeutika haben eine Wirkung in diesem Modell gezeigt, zumindest wenn sie vor dem toxisch bedingten Gewebeumbau angewendet wurden.

MCT (60mg/kg) wurde bei männlichen Sprague-Dawley Ratten als Einzelinjektion subkutan verabreicht und ein gleiches Volumen isotonischer Kochsalzlösung bei den Kontrolltieren injiziert. An den Tagen 14-28 des Experiments wurde entweder 0.25 mg/kg Lisurid oder 2.5 mg/kg Tergurid pro Tag über eine Magensonde verabreicht. Dabei wurde die genannte Dosis der jeweiligen Testsubstanz morgens und abends in einem Volumen von je 2.0 ml an Gruppen von jeweils 6 Tieren gegeben, die an Tag 1 des Experiments mit MCT behandelt worden waren. Die gleiche Menge Wasser wurde den Kontrolltieren zugeführt.

Am Tag 28 des Experiments, zwei Stunden nach letztmaliger Substanzgabe, wurden die Tiere narkotisiert unter Verwendung von Pentobarbital. Eine Tracheotomie wurde durchgeführt und die Tiere unter Isofluran-Anästhesie beatmet. Der mittlere arterielle Systemdruck und der systolische Druck im rechten Herzventrikel wurden gemessen. Nach den Druckmessungen wurden die Tiere mit physiologischer Kochsalzlösung perfundiert und die rechte Lunge wurde zur Bestimmung des Kollagengehalts entfernt.

Die sc-Injektion von MCT, wie oben beschrieben, führt zu einer schweren Schädigung des pulmonalen Gefässendothels sowie überschießender Produktion von Bindegewebe und Entwicklung einer pulmonalen Hypertonie. Anhäufung von Kollagen, gemessen als Hydroxyprolin-Gehalt des Lungengewebes, und Anstieg des rechtsventrikulären systolischen Drucks reflektieren die beschriebenen strukturellen und funktionalen Veränderungen. Die möglichen therapeutischen und präventiven Effekte von Lisurid bzw. Tergurid wurden in dem vorgestellten Modell der pulmonalen Hypertension gemessen. Dabei wurde unter den Bedingungen des beschriebenen Experiments die Behandlung mit Lisurid respektive Tergurid nicht zum Zeitpunkt der experimentell gesetzten Gewebeschädigung begonnen, sondern erst 14 Tage nach Eintritt des Gewebeschadens. Laut vorhandener Literatur zum experimentellen Modell haben sich zu diesem Zeitpunkt bereits ausgeprägte Gefässveränderungen und ein Anstieg des rechtsventrikulären Drucks manifestiert.

Wie die Tabellen 1 und 2 zeigen, reduzieren Lisurid und Tergurid im Sinne eines therapeutisch erwünschten Effekts den pathologischen Druckanstieg im rechten Ventrikel als Maßstab des erhöhten Pulmonaldrucks. Als strukturelles Korrelat wurde unter Behandlung mit Lisurid und Tergurid eine Senkung des MCT-induzierten Anstiegs im Hydroxyprolingehalt der Lunge gemessen, im Sinne eines "reverse remodeling".

**Tabelle 1: Einfluss einer Behandlung mit Lisurid oder Tergurid an den Tagen 15-28 des Experiments auf den systolischen rechtsventrikulären Druck (RVPsys) und auf den arteriellen Systemdruck (SAP)**

| | RVPsys (mmHG) | SAP (mmHG) |
|---|---|---|
| Control | 23 ± 4 | 118 ± 5 |
| Monocrotaline | 55 ± 5 | 114 ± 7 |
| Monocrotaline + 0.25 mg/kg Lisuride bid | 43 ± 7 | 109 ± 9 |
| Monocrotaline + 2.5 mg/kg Terguride bid | 39 ± 3 | 111 ± 7 |

Ergebnisse als Durchschnitt ± SEM (N=6)

**Tabelle 2: Einfluss einer Behandlung mit Lisurid oder Tergurid an den Tagen 15-28 des Experiments auf den Hydroxyprolingehalt der Lunge**

| | Hydroxyproline (ug/g protein) |
|---|---|
| Control | 1.2 ± 0.2 |
| Monocrotaline | 4.2 ± 1.1 |
| Monocrotaline + 0.25 mg/kg Lisuride bid | 3.3 ± 0.8 |
| Monocrotaline + 2.5 mg/kg Terguride bid | 2.7 ± 1.2 |

Ergebnisse als Durchschnitt ± SEM (N=6)

Bewertung der experimentellen Ergebnisse: Es kann angenommen werden, dass im Monocrotalin-Modell vaskuläre Effekte feststellbar sind (z.B. von Prostanen) wie sie für die Therapie des pulmonalen Hochdrucks eingesetzt werden können. Das Modell gilt aber nicht als spezifisch für diesen Wirkungsmechanismus, wie er primär über die Kapillaren oder Arteriolen der Lunge vermittelt wird. Vielmehr findet sich in diesem Modell auch eine stark vermehrte Bildung von Kollagen, das von sich vermehrenden Fibroblasten und Fibrozyten, d.h. Zellen des Mesenchyms, produziert wird. Dies zeigt sich im Anstieg des Hydroxyprolin-Spiegels der Lunge nach Hydrolyse (weitestgehend aus Kollagen entstanden) und in der Hemmung dieses Anstiegs durch Vorbehandlung mit 5-HT2B-Antagonisten wie Lisurid und Tergurid, wie im Experiment gezeigt. Dies spricht für ein zumindest partielles "reverse modeling", d.h. eine Wiederherstellung normaler Lungenstrukturen.

Der gleiche "reverse modeling"-Effekt ist im Experiment auch in der rechten Herzkammer feststellbar und insgesamt kommt es dadurch zu einem Abfall des pathologisch erhöhten pulmonalen Drucks. Dabei ist der erzielte Abfall/Normalisierung des erhöhten Drucks in der Lunge nicht, wie beim primären pulmonalen Hochdruck, eine erste Folge von Gefäßeffekten wie z.B. nach Anwendung von Prostanen. Vielmehr ist der beobachtete therapeutische Effekt im Fall der Anwendung der 5-HT2B-Antagonisten in erster Linie die Folge einer Hemmung der vorwiegend durch 5-HT induzierten mesenchymalen Zellproliferation. Diese überraschende Erkenntnis wird bestätigt durch die Feststellung einer sofortigen Hemmung des durch 5-HT induzierten Einbaus von 3H-Thymidin, einem etablierten Marker von Zellteilung und Proliferation, durch Substanzen wie Lisurid und Tergurid (in Beispiel 1 beschrieben). Einem möglichen Effekt dieser Substanzen auf Blutkapillaren oder Arteriolen des Lungengewebes kommt hier nur eine nachrangige Funktion zu.

Diese neue Erkenntnis erlaubt es jetzt, derartige Substanzen auch bei nicht vaskulär verursachten Formen von PAH und anderen durch exzessive 5-HT2-induzierte Proliferation ausgelösten Organerkrankungen einzusetzen und dabei wieder normale Strukturen und Funktionen herzustellen. Bestätigt wird dies durch Ergebnisse der Gruppe von Launay, die zeigen konnte, dass eine exzessiv gesteigerte 5-HT-Aktivität pränatal , d.h. während der Ontogenese, in transgenen Mäusen mit Überexpression von 5-HT2B-Rezeptoren zu schweren Störungen in der Struktur des Herzens führt. [Nebigil CG *et al.,* 2001] Daraus ist zu schließen, dass auch hier ein "Remodeling" durch 5-HT2B-Antagonisten von großer Bedeutung für die Behandlung solcher pathologischer Strukturen sein kann. Ein analoger ursächlicher Zusammenhang zu verstärkter 5-HT-Wirkung gilt möglicherweise auch für beobachtete Herzmissbildungen bei Neugeborenen von Frauen, die wegen einer Schwangerschaftsdepression mit 5-HT-Wiederaufnahme-Hemmern behandelt wurden. Auch hier legen die beobachteten Herzmissbildungen Neugeborener nahe, dass die Befunde einer pathologischen Stimulierung von 5-HT2B-Rezeptoren sind.

### Beispiel 4

### Antioxidative Wirkung von Lisurid

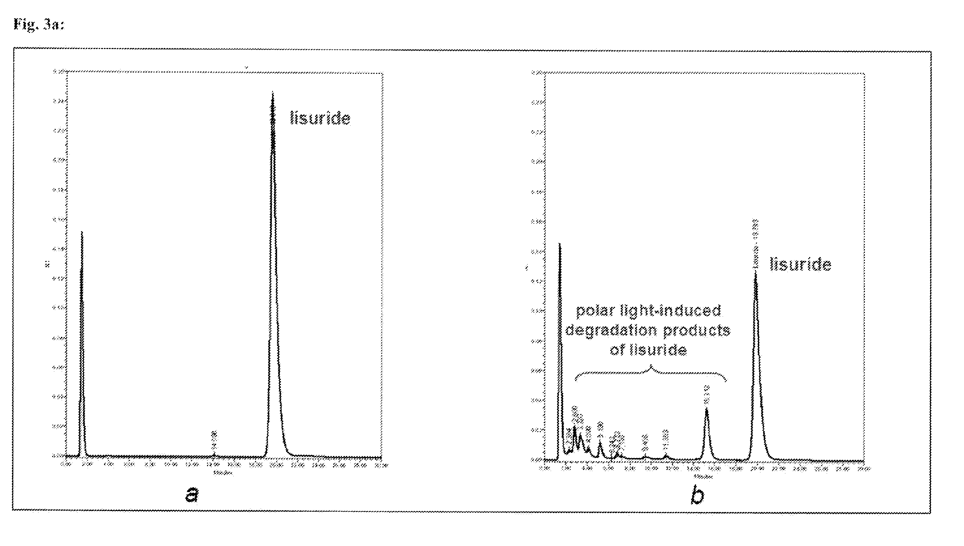

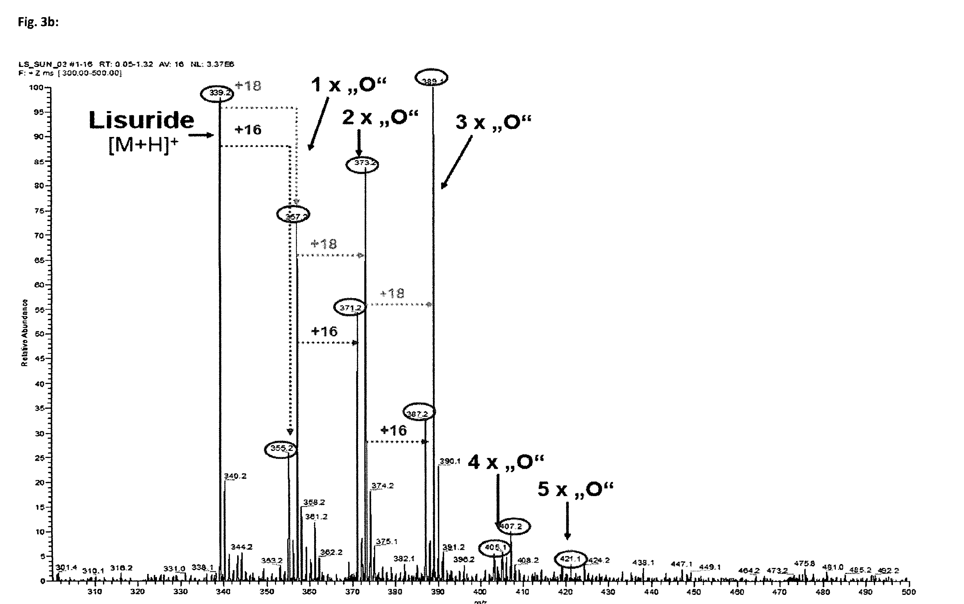

Löst man Lisurid in Wasser, so wird bereits bei Raumtemparatur wird gemessen an den Retentionszeiten im HPL-Chromatogramm (Fig. 3A) gelöstes Lisurid schnell zu Produkten abgebaut, die eine höhere Polarität aufweisen als die Ausgangssubstanz.

Lisurid durchläuft in Lösung und unter Lichteinfluss verschiedene Reaktionen, insbesondere kommt es zur Bindung von Sauerstoff-Radikalen. Die massenspektroskopisch in der Mehrheit nachweisbaren Reaktionsprodukte enthalten 2 Sauerstoffatome; es lassen sich aber auch Verbindungen mit 3, 4 und 5 Sauerstoff-Atomen nachweisen.

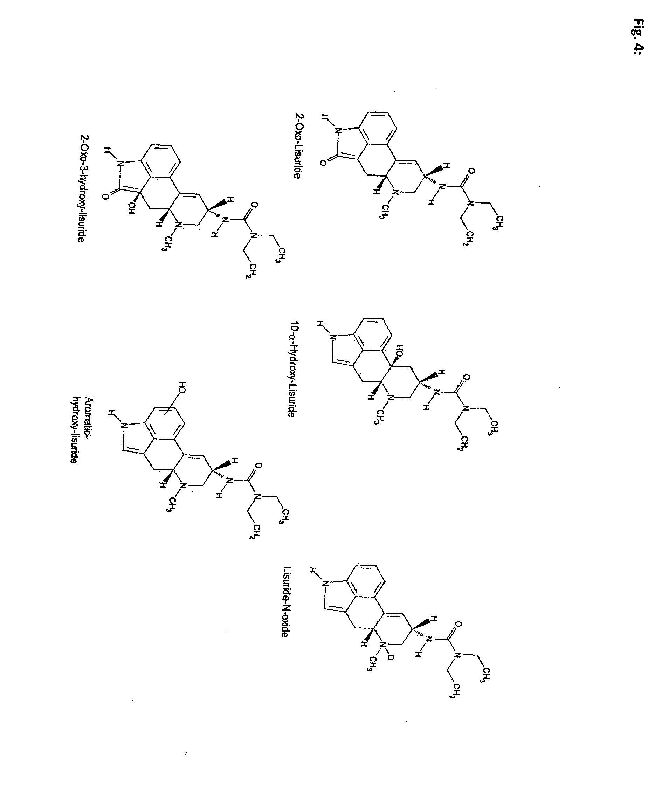

Ein typisches Charakteristikum von Massenspektra ist, dass Gipfel von [M+H]++18, d. h. Zusatz von Wasser, oder [M+H]++16, d.h. Zusatz eines Sauerstoffatoms, gefunden werden, wie auch Kombinationen von Wasser und Sauerstoffatomen. Wegen der niedrigen in den Entnahmeproben enthaltenen Substanzmengen und der Kurzlebigkeit der Reaktionsprodukte war deren Isolierung zur Strukturaufklärung nicht möglich. Das Lisuridmolekül weist mehrere Positionen auf, an denen Wasser und/oder ein Sauerstoffatom gebunden werden können (Fig. 4).

Die zuvor beschriebenen Ergebnisse können von großer Bedeutung sein, da gelöstes Lisurid ein starker Radikalfänger ist. Wie bereits weiter oben ausgeführt, wurde in Untersuchungen zur 5-HT2-induzierten Herzhypertrophie nachgewiesen, dass dieser Prozess unter Generierung von Sauerstoffradikalen abläuft [Bianchi P *et al.,* 2005]. Wenn man weiter berücksichtigt, dass diese Substanzen durch ihre bisher nie erreichte hohe 5-HT2-Rezeptoraffinität bevorzugt an diesen angereichert werden (die wiederum bei Organhypertrophie lokal verstärkt exprimiert sind) so trägt auch diese Eigenschaft wesentlich zu einer Hemmung pathologischen Organwachstums bei.

### Beispiel 5

### Herstellung eines sterilen Lyophilisates mit Lisurid zur Injektion nach Auflösung.

1.0 g Lisurid Hydrogenmaleat wird mit 20 g Laktose-Monohydrat, 0,4g Zitronensäure-Monohydrat und 1 g Natriumcitrat-Dihydrat in 977,6 g Wasser für Injektionszwecke aufgelöst. Die farblose Lösung, welche einen pH-Wert zwischen 4,5 und 5 aufweist, wird anschließend durch einen Membranfilter und danach durch einen Sterilfilter (0,2µm) unter aseptischen Bedingungen filtriert und zu jeweils 1 g in geeignete Vials verfüllt. Nach Verschließen mit einem geeigneten Stopfen wird die Lösung bei minus 40-50°C eingefroren und anschießend im Vakuum unter Verwendung eines geeigneten Gefriertrockners getrocknet wobei im Vial ein Trockenkuchen aus den Formulierungbestandteilen entsteht. Danach werden die Vials verschlossen. Auf diesem Weg entsteht ein Batch mit 1.000 Vials (theoretische Ausbeute) mit einer Einzeldosis von 1 mg Lisurid Hydrogenmaleat. Das so erhaltene Lyophilisat kann zum Beispiel mit steriler physiologischer Kochsalzlösung im Vial rekonstituiert werden und ergibt eine anwendungsgerechte Lösung zur Injektion zur sofortigen Anwendung, wobei die Zusammensetzung der Lösung mit den ausgewählten Hilfsstoffen eine ausreichende Stabilität unter Anwendungsbedingungen von mindestens 24 h ergibt.

### Beispiel 6

### Herstellung eines sterilen Lyophilisates mit Tergurid zur Injektion nach Auflösung.

2.0 g Tergurid wird mit 20 g Laktose-Monohydrat, 0,4 g Zitronensäure- Monohydrat und 1 g Natriumcitrat-Dihydrat in 976,6 g Wasser für Injektionszwecke aufgelöst. Die farblose Lösung, welche einen pH-Wert zwischen 4,5 und 5 aufweist, wird anschließend durch einen Membranfilter und danach durch einen Sterilfilter (0,2µm) unter aseptischen Bedingungen filtriert und zu jeweils 1 g in geeignete Vials verfüllt. Nach Verschließen mit einem geeigneten Stopfen wird die Lösung bei minus 40-50°C eingefroren und anschießend im Vakuum unter Verwendung eines geeigneten Gefriertrockners getrocknet, wobei im Vial ein Trockenkuchen aus den Formulierungbestandteilen entsteht. Danach werden die Vials verschlossen. Auf diesem Weg entsteht ein Batch mit 1.000 Vials (theoretische Ausbeute) mit einer Einzeldosis von 2 mg Tergurid. Das so erhalteneLyophilisat kann z. B. mit steriler physiologischer Kochsalzlösung im Vial rekonstituiert werden und ergibt eine anwendungsgerechte Lösung zur Injektion zur sofortigen Anwendung, wobei die Zusammensetzung der Lösung mit den ausgewählten Hilfsstoffen eine ausreichende Stabilität unter Anwendungsbedingungen von mindestens 24 h ergibt.

### Beispiel 7

### Herstellung eines Matrixpflasters mit Tergurid für die transdermale Anwendung

2.5 g Tergurid wird gelöst in 2,13 g Acetone und 51,54 g einer Lösung von basischem Buty-Methacrylat-Coplymer (Eudragit E 100 Lösung). 5 g Polyvinylpyrrolidon (Povidon 25), 2,5 g Propylenglycol, 5 g Dodecyl(-N,N-dimethlyaminoacetat, alternativ 1-Dodecanol-n-alkylether), 1 g Foral E 105 und 0,65 g Antioxidans (Butylhydroxyanisol oder Vitamin E) werden der Lösung zugesetzt. Die so erhaltene viskose Lösung wird kontinuierlich auf eine Polymerfolie zum Beispiel aus Polyethylen geschichtet und unter geeigneten Prozeßbedingungen unter Entfernung der flüchtigen Lösungsmittel getrocknet bis zu einem Flächengewicht von ca. 50 mg/ 10cm2 (±5%). Diese klebende Matrix wird laminiert mit einer weiteren Polymerfolie zum Beispiel aus Polyethylenterephtalat, welche einseitig silikonisiert ist und anschließend in für die therapeutische Anwendung geeignete einzelne Pflaster von 10 oder 20 cm2 gestanzt und luft- und feuchtigkeitsgeschützt verpackt. Ein so hergestelltes Tergurid Pflaster setzt den eingearbeiteten Wirkstoff kontinuierlich über mehrere Tage mit einer Freisetzungsrate zwischen 0,1 bis 0,5 µg/cm2/h an die intakte Humanhaut frei.

### Beispiel 8

### Herstellung eines Matrixpflasters mit Lisurid für die transdermale Anwendung

2.5 g Lisurid wird gelöst in 2,13 g Acetone und 51,54 g einer Lösung von basischem Buty-Methacrylat-Coplymer (Eudragit E 100 Lösung). 5 g Polyvinylpyrrolidon (Povidon 25), 5 g Propylenglycolmonolaurat (PGML), (alternativ PGML/Eutanol® (2-Octyldodecanol) 10:1 oder PGML/Transcutol® (Diethylenglycolmonoethylether) 10:1), 1 g Foral E 105 und 0,65 g Antioxidans (Butylhydroxyanisol oder Vitamin E) werden der Lösung zugesetzt. Die so erhaltene viskose Lösung wird kontinuierlich auf eine Polymerfolie z. B. aus Polyethylen geschichtet und unter geeigneten Prozeßbedingungen unter Entfernung der flüchtigen Lösungsmittel getrocknet bis zu einem Flächengewicht von ca. 50 mg/ 10cm2 (±5%). Diese klebende Matrix wird laminiert mit einer weiteren Polymerfolie z. B. aus Polyethylenterephtalat, welche einseitig silikonisiert ist und anschließend in für die therapeutische Anwendung geeignete einzelne Pflaster von 5 oder 10 cm2 gestanzt und luft-und feuchtigkeitsgeschützt verpackt. Ein so hergestelltes Lisurid Pflaster setzt den eingearbeiteten Wirkstoff kontinuierlich über mehrere Tage mit einer Freisetzungsrate zwischen 0,1 bis 0,5 µg/cm2/h an die intakte Humanhaut frei.

### Beispiel 9

### Herstellung einer sterilen Zubereitung mit Tergurid zur subkutanen Anwendung als Implantat.

50 g mikronisiertes Tergurid wird homogen mit 50 g Polydimethylsiloxan gemischt und die Mischung mittels geeigneter Standardverfahren vorzugweise durch Extrusion in eine fadenförmige Matrix ausgezogen, welche in Stücke zu 30 mm geteilt wird. Eine wirkstofffreie Matrix wird in gleicher Wiese hergestellt. In einem zweiten Schritt wird eine tubenförmige Membran mit einer Wandstärke von zum Beispiel 0,2 mm ebenfalls durch Extrusion aus kommerziell erhältlichem Polydimethylsiloxan, welches Siliciumdioxid enthält oder unter Verwendung von zum Beispiel Polydimethylsiloxan, welches mit Platin katalysiertes vernetztes Polyalkenoxid (Gelest®) enthält, hergestellt. Diese Membranen werden in Stücke von jeweils 60 mm Länge abgeteilt und in Cyclohexan quellen lassen. Die wirkstoffhaltige Matrix wird in die tubenförmige Membran eingefügt ebenso wie die wirkstofffreie Matrix, geeigneter Länge an beiden Enden der tubenförmigen Membran in einer Weise, dass je ein Luftraum von ca.1-3 mm an beiden Seiten zwischen der wirkstoffhaltigen und den wirkstofffreien Matrizen bestehen bleibt. Cyclohexan wird durch Verdunsten entfernt, die Formulierung auf ein Gesamtlänge von 50 mm geschnitten und an den Enden verschmolzen. Das Produkt wir mit Gas (H2O2 oder Ethylenoxid) sterilisiert und in geeigneter Weise verpackt. SO entsteht ein Implantat zur Applikation unter der Haut, welches durch die Lufteinschlüsse mittels Ultraschalldetektion in vivo lokalisiert werden kann.

### Beispiel 10

### Herstellung einer sterilen Zubereitung mit Lisurid zur subkutanen Anwendung als Implantat.

10 g mikronisiertes Tergurid wird homogen mit 90 g Polydimethylsiloxan gemischt und die Mischung mittels geeigneter Standardverfahren vorzugweise durch Extrusion in eine fadenförmige Matrix ausgezogen, welche in Stücke zu 30 mm geteilt wird. Eine wirkstofffreie Matrix wird in gleicher Wiese hergestellt. In einem zweiten Schritt wird eine tubenförmige Membran mit einer Wandstärke von zum Beispiel 0,2 mm ebenfalls durch Extrusion aus kommerziell erhältlichem Polydimethylsiloxan, welches Siliciumdioxid enthält oder unter Verwendung von zum Beispiel Polydimethylsiloxan, welches mit Platin katalysiertes vernetztes Polyalkenoxid (Gelest®) enthält, hergestellt. Diese Membranen werden in Stücke von jeweils 60 mm Länge abgeteilt und in Cyclohexan quellen lassen. Die wirkstoffhaltige Matrix wird in die tubenförmige Membran eingefügt ebenso wie die wirkstofffreie Matrix, geeigneter Länge an beiden Enden der tubenförmigen Membran in einer Weise, dass je ein Luftraum von ca.1-3 mm an beiden Seiten zwischen der wirkstoffhaltigen und den wirkstofffreien Matrizen bestehen bleibt. Cyclohexan wird durch Verdunsten entfernt, die Formulierung auf ein Gesamtlänge von 50 mm geschnitten und an den Enden verschmolzen. Das Produkt wir mit Gas (H2O2 oder Ethylenoxid) sterilisiert und in geeigneter Weise verpackt. SO entsteht ein Implantat zur Applikation unter der Haut, welches durch die Lufteinschlüsse mittels Ultraschalldetektion in vivo lokalisiert werden kann.

### Beispiel 11

Affinitäten von Lisurid und verwandten Verbindungen zu 5-HT_{2B} Rezeptoren von Schweinelungenarterien und bei 5-HT_{2A} Rezeptoren von Schweineherzkranzarterien.

| | 5-HT_{2B} | | 5-HT_{2A} | |
|---|---|---|---|---|
| Verbindung | pA₂ | *n* | pA₂ | *n* |
| Lisurid | 10.32 ± 0.10^{b} | 4 | 9.40 0.05^{a,c} | 4 |
| *N"-*Monodesethyllisurid | 8.08 ± 0.05 | 6 | 8.29 ± 0.03 | 5 |
| 6-Norlisurid | 8.39 ± 0.12 | 4 | 8.84 ± 0.04 | 4 |
| 8β-Lisurid | 9.27 ± 0.11 | 4 | 8.54 ± 0.04 | 9 |
| Tergurid | 8.87 ± 0.06 | 9 | 9.43 ± 0.08^{a} | 6 |
| *N"-*Monodesethyltergurid | 7.30 ± 0.02 | 4 | 7.82 ± 0.06 | 5 |
| 6-Nortergurid | 7.11 ± 0.08 | 4 | 7.85 ± 0.04 | 5 |
| 8β-Tergurid | 8.39 ± 0.09 | 4 | 8.29 ± 0.03 | 5 |
| Oxtergurid | 6.42 ± 0.20 | 4 | 6.99 ± 0.04 | 4 |
| Ketanserin | - | | 8.88 ± 0.03^{c} | |
| SB204741 | 6.59 ± 0.20^{d} | 4 | - | |

a= pD'2 -Wert (negativer dekadischer Logarithmus einer Antagonisten-Konzentration, die den Maximal-Effekt um 50% reduziert; wird bei nicht-kompetitiven Antagonismus verwendet im Gegensatz zum pA2 (=negativer dekadischer Logarithmus der Antagonisten-Konzentration, die es erforderlich macht, die Agonisten-Konzentration zu verdoppeln, um den ursprünglichen Agonisten-Effekt wiederherzustellen), b und c= Werte aus Diss. Jähnichen, FU Berlin, 2005, d= von Glusa, E und Pertz, H H, Brit. J. Pharmacol.130, 692-698, 2000. Ketanserin ist ein Standard-5HT2A-Antagonist, SB204741 ein Standard-5-HT2B-Antagonist. Experimentelle Bedingungen wie bei Beispiel 1.

### REFERENZEN

1. Bianchi P, Pimentel DR, Murphy MP, Colucci WS, Parini A.(2005) A new hypertrophic mechanism of serotonin in cardiac myocytes: receptor-independent ROS generation. FASEB J. 19, 641-643.
2. Glusa E, Markwardt F (1984) Interaction of lisuride with monoamine receptors on human blood platelets. Biochem. Pharmacol. 1984; 33: 493-496
3. Hauso O, Gustafsson BI, Loennechen JP, Stunes AK, Nordrum I, Waldum HL (2007) Long-term serotonin effects in the rat are prevented by terguride. Regulatory Peptides 143 (2007) 39-46
4. Hofmann, C., Penner, U., Dorow, R., Pertz, H.H., Jähnichen, S., Horowski, R., Latte, K.P., Palla, D., Schurad, B. (2006) Lisuride, a Dopamine receptor Agonist with 5-HT2B Receptor Antagonist Properties: Absence of Cardiac Valvulopathy Adverse Drug Reaction Reports Supports the Concept of a Crucial Role for 5-HT2B Receptor Agonism in Cardiac Valvular Fibrosis. Clin Neuropharmacol 2006; 29: 80-86
5. Jähnichen S, Horowski R, Pertz HH (2005) Agonism at 5-HT2B receptors is not a class effect of the ergolines Eur J Pharmacol 2005; 513: 225-228
6. Nebigil, C.G., Hickel, P., Messaddeq, N., Vonesch, J-L., Douchet, M.P., Monassier, L., György, K., Matz, R., Andriantsitohaina, R., Manivet, Ph., Launay, J.-M., Maroteaux, L. (2001) Ablation of Serotonin 5-HT2B Receptors in Mice Leads to Abnormal Cardiac Structure and Function Circulation 2001; 103: 2973-2979
7. Redout EM, van der Torn A, Zuidwijk MJ, van de Kolk CW, van Echtheld CJA, Musters RJP, van Hardeveld C, Paulus WJ, Simonides WS (2010) Antioxidant treatment attenuates pulmonary arterial hypertension-induced heart failure. Am J Physiol Heart Circ Physiol 298, H1038-H1047
8. Podvalovä I, Dlaba 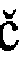 A (1972) Lysenyl, a new antiserotonin agent Res. clin. Stud. Headache 1972; 3: 325-334
9. Reiter R, Horowski R, Tack J (2007) Pharmaceutical compositions for the treatment of capillary arteriopathy. US patent appl publ No. US 2009/0325997 A1
10. Stocchi F, Ruggieri S, Vacca L, Olanow CW (2002) Prospective randomized trial of lisuride infusion versus oral levodopa in patients with Parkinson's disease Brain 2002; 125: 2058-2066
11. Ulrich-Somaini S (2009) Management der pulmonalen Hypertonie - was ist neu seit Dana Point? Kardiovaskuläre Medizin 2009;12(9):245-250
12. Villalon, C.M., Centurion, D., Valdivia, L.F., de Vries, P., Saxena P.R. (2003) Migraine : pathophysiology, pharmacology, treatment and future trends, Curr vasc Pharmacol 2003; 1: 71-84
13. Villeneuve C, Caudrillier A, Ordener C, Pizzinat N, Parini A, Mialet-Perez J.(2009) Dose-dependent activation of distinct hypertrophic pathways by serotonin in cardiac cells. Am.J.Physiol Heart Circ.Physiol 297, H821-H828.

### FIGURENBESCHREIBUNG

Fig. 1: Antagonistische Effekte von Lisurid und Tergurid auf (A) 5-HT-induzierte und 5-HT2B-mediierte Relaxierung von PGF2α-vorkontrahierten Pulmonalarterien des Schweins und auf die (B) 5-HT-induzierte Kontraktion von 5-HT2A-mediierten Koronararterien des Schweins [Jaehnichen S *et al.* 2005]
Fig. 2: Reduktion der Zellproliferation unter Tergurid und Lisurid
Fig. 3A: HPL-Chromatogramme a. sofort nach Lösung von Lisurid Hydrogenmaleat in Wasser (0.4 mg/ml) and b. nach 4 Stunden bei Raumtemperatur und Tageslicht.
Fig.3B: Massenspektrum einer wässrigen Lösung von Lisurid 5 Stunden nach Lichtexposition; es werden Beispiele der [M+H]++16 and [M+H]++18-Schritte für Wasser sowie ein oder mehrere zusätzliche Sauerstoffatome gegeben.
Fig. 4: Bindungsstellen im Lisuridmolekül, an denen Wasser oder Sauerstoffatome angelagert werden können und Strukturbeispiele.

## Patentansprüche

1. Lisurid, Tergurid oder ein Derivat der allgemeinen Formel (I): 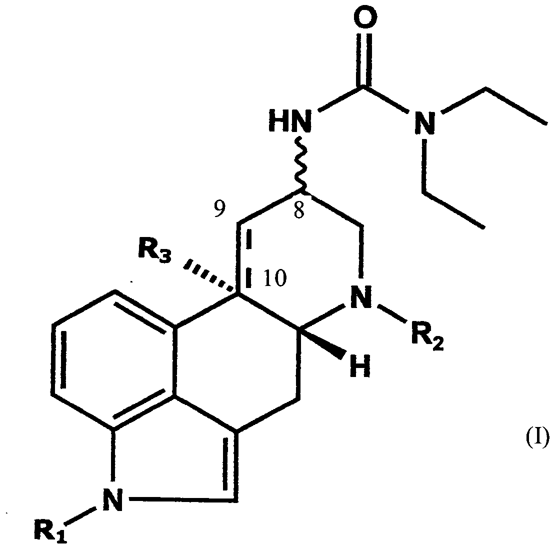
R1: allyl, alkinyl
R2: ethyl, n-propyl, i-propyl, allyl
R3: hydrogen, methyl, ethyl, n-propyl, i-propyl,-CH2OH
wobei die Bindung zwischen C9/C10 entweder eine Einfach-
oder eine Doppelbindung ist,
zur Verwendung in der Prophylaxe und/oder Therapie fibrotischer Veränderungen von Organen und deren Gefäßstruktur in einem menschlichen oder tierischen Lebewesen zum Aufhalten und / oder zur Rückbildung dieser fibrotischen Veränderungen von Organen und deren Gefäßstruktur.

2. Lisurid oder Tergurid oder ein Derivat der allgemeinen Formel (I) zur Verwendung in der Prophylaxe und/oder Therapie gemäß Anspruch 1 zur Lebensverlängerung des Lebewesens.

3. Lisurid oder Tergurid oder ein Derivat der allgemeinen Formel (I) zur Verwendung in der Prophylaxe und/oder Therapie gemäß Anspruch 1 oder 2 wobei während der Therapiezeit mindestens 80 % der Zeit, bevorzugt mindestens 100 % der Therapiezeit, die 5-HT_{2B}- und/oder 5-HT_{2A}-Rezeptor Belegung im Zielorgan wenigstens 90 % beträgt.

4. Lisurid oder Tergurid oder ein Derivat der allgemeinen Formel (I) zur Verwendung in der Prophylaxe und/oder Therapie gemäß Anspruch 3 wobei während der gesamten Therapiezeit die 5-HT_{2B}- und/oder die 5-HT_{2A}-Rezeptor Belegung im Zielorgan vollständig ist.

5. Lisurid oder Tergurid oder ein Derivat der allgemeinen Formel (I) zur Verwendung in der Prophylaxe und/oder Therapie gemäß einem der Ansprüche 1 bis 4 wobei der Wirkstoffspiegel in der systemischen Zirkulation des Lebewesens während der Therapiezeit mindestens 80 % der Zeit, bevorzugt 100 % der Zeit (kontinuierlich) wenigstens 5 pg/ml, mehr bevorzugt wenigstens 100 pg/ml, mehr bevorzugt wenigstens 200 pg/ml, am meisten bevorzugt 300 - 500 pg/ml beträgt.

6. Lisurid oder Tergurid oder ein Derivat der allgemeinen Formel (I) zur Verwendung in der Prophylaxe und/oder Therapie gemäß einem der Ansprüche 1 bis 5 wobei die Verabreichung in einer Dosis von 0,01 bis 5,0 mg pro Tag, bevorzugt von 0,15 bis 3,0 mg pro Tag am meisten bevorzugt von 0,25 bis 1,0 mg pro Tag erfolgt.

7. Lisurid oder Tergurid oder ein Derivat der allgemeinen Formel (I) zur Verwendung in der Prophylaxe und/oder Therapie gemäß einem der Ansprüche 1 bis 6 wobei die Verabreichung kontinuierlich erfolgt.

8. Lisurid oder Tergurid oder ein Derivat der allgemeinen Formel (I) zur Verwendung in der Prophylaxe und/oder Therapie gemäß einem der Ansprüche 1 bis 7, wobei die Verabreichung kontinuierlich in einer Tages-Dosis von 0,01 bis 5,0 mg , bevorzugt von 0,15 bis 3,0 mg am meisten bevorzugt von 0,25 bis 2,0 mg erfolgt.

9. Lisurid oder Tergurid oder oder ein Derivat der allgemeinen Formel (I) zur Verwendung in der Prophylaxe und/oder Therapie gemäß einem der Ansprüche 1 bis 8, wobei das Lebewesen an erhöhtem pulmonaren Gefäßdruck (PAH) leidet.

10. Lisurid oder Tergurid oder ein Derivat der allgemeinen Formel (I) zur Verwendung in der Prophylaxe und/oder Therapie gemäß Anspruch 9, wobei der PAH Folge einer Erkrankung ist ausgewählt aus der Gruppe enthaltend COPD, Infektionen, Rechtsherzhypertrophie, -insuffizienz als Folge pulmonaler Hypertension, sowie sonstige fibrotische Veränderungen der Lunge, Leber, Niere, Haut oder anderer Organsysteme.

11. Pharmazeutische Zubereitung enthaltend Lisurid, oder Tergurid oder oder ein Derivat der allgemeinen Formel (I) zur Verwendung in der Prophylaxe und/oder Therapie gemäß einem der Ansprüche 1 bis 10.

12. Pharmazeutische Zubereitung gemäß Anspruch 11, mit einer Einzeldosis von Lisurid oder Tergurid oder oder ein Derivat der allgemeinen Formel (I) im Bereich von 0,01 bis 25 mg, bevorzugt im Bereich von 0,15 bis 3,0 mg am meisten bevorzugt im Bereich von 0,25 bis 2,0 mg erfolgt.

13. Pharmazeutische Zubereitung gemäß Anspruch 11 oder 12 wobei die Formulierung ausgewählt ist aus der Gruppe enthaltend Tabletten, Schichttabletten, überzogenen Tabletten, Pillen, Weich- oder Hartkapseln,
Mikrokapseln, orale Retardarzneiformen, transdermalen Systeme, Suppositorien, micro- und nanokristallinen Formulierungen, liposomalen Formulierungen, Tropfen, Nasentropfen, Sprays, Emulsionen, Dispersionen, Lösungen, sterile Lösungen Lyophilisaten, Pulver und Inhaltionssprays.

14. Pharmazeutische Zubereitung gemäß einem der Ansprüche 11 bis 13, wobei die Applikation oder Anwendung ausgewählt ist aus der Gruppe enthaltend orale, perorale, sublinguale, buccale, subcutane, intravenöse dermale, pulmonale oder nasale Anwendung bzw. Applikation, wobei eine subcutane Anwendung am meisten bevorzugt ist

15. Pharmazeutische Zubereitung gemäß einem der Ansprüche 11 bis 14, wobei eine sterile Lösung entweder als Lyophilisat zur Zubereitung einer sterilen Lösung vor der Anwendung oder als gebrauchsfertige sterile Lösung mit einer Dosierung von 0,25 bis 1,0 mg für die kontinuierliche vorzugsweise subcutane Infusion mit einer Infusionsrate von 0,05 bis 50 mcg/h vorzugsweise 1 bis 20 mcg/h am meisten bevorzugt ist.
